# EUROPEAN PATENT APPLICATION

(11) **EP 2 453 019 A1**
(43) Date of publication of application: **16.05.2012**
(21) Application number: 10796763.0
(22) Date of filing: 09.07.2010
(51) Int. Cl.: C12N 15/16, C12N 15/85, A61K 48/00, A61P 3/08

(54) **GENE THERAPY COMPOSITIONS FOR PREVENTING AND/OR TREATING AUTOIMMUNE DISEASES**

(30) Priority: 10.07.2009 ES 200930442
(71) Applicant: Universidad Autònoma de Barcelona, 08193 Barcelona (ES)
(72) Inventor: ANGUELA MARTÍNEZ, Xavier, E-08193 Bellaterra - Barcelona (ES); BOSCH TUBERT, María Fátima, E-08193 Bellaterra - Barcelona (ES); TARUFO, Sabrina, E-08193 Bellaterra - Barcelona (ES)
(74) Representative: Illescas, Manuel
(86) International application number: PCT/ES2010/070478
(87) International publication number: WO 2011/004051

(57) **Abstract**

Gene therapy compositions for the prevention and/or treatment of autoimmune diseases. The present invention relates to vectors, preferably plasmids that comprise specific promoters, that encode insulin-like growth factor I (IGF-I) or a functional biological equivalent thereof, which will be used to prevent or treat Type 1 Diabetes (DT1) and/or other autoimmune diseases. Moreover, the invention comprises the method of preventing or treating DT1 by means of the expression of IGF-I, or a functional biological equivalent thereof, in hepatic cells. IGF-I was capable of interrupting the immune attack against pancreatic β cells, thereby preventing the loss of β cell mass and, consequently, maintaining normal levels of circulating insulin.

## Description

### FIELD OF THE INVENTION

The present invention relates to gene therapy compositions that comprise vectors with at least one sequence that encodes insulin-like growth factor 1 (IGF-I), which will be used to prevent or treat autoimmune diseases. Moreover, the invention comprises the method of preventing or treating type 1 diabetes (DT1) by the expression of IGF-I, specifically in hepatic cells. IGF-I was capable of stopping the immune attack against pancreatic β cells, thereby preventing the loss of β cell mass and, consequently, maintaining circulating insulin at normal levels.

Therefore, the present invention belongs to the field of gene therapy, in particular the gene therapy that focuses on the prevention or treatment of autoimmune diseases.

### STATE OF THE ART

DT1 is the result of the autoimmune destruction of pancreatic β cells, which leads to insulin deficiency and hyperglycemia (Eizirik et al., 2009). The onset of spontaneous DT1 in human beings is preceded by a progressive leukocyte infiltration in the islets (insulitis), which persists for a relatively long period of time prior to the massive destruction of islet β cells (Atkinson and Eisenbarth, 2001). DT1 is diagnosed when the destruction of β cells is almost complete (Couper et al., 1991) and patients need insulin replacement therapy to survive. Therefore, preventive interventions aimed at interrupting the immune attack against the islets and preventing the loss of β cells are of great interest.

Several growth factors have shown protective properties against DT1 in different animal models. Amongst them, IGF-I is an interesting candidate for an anti-diabetes gene for several reasons. In the first place, it has anti-apoptotic effects *in vitro* in islets exposed to proinflammatory cytokines (Giannoukakis et al., 2000). In the second place, the daily subcutaneous administration of recombinant human IGF-1 to pre-diabetic NOD (non-obese diabetic) mice reduces the severity of insulitis and the incidence of DT1, which demonstrates the efficacy of the protection of IGF-I against DT1. However, there is a marked adverse effect minutes after the administration of free IGF-I: the circulating levels of glucose decrease, causing lethal hypoglycemia in some animals. This adverse effect is due to the insulin-like hypoglycemic action of IGF-I. Hypoglycemia and its potentially lethal resulting adverse effects, jointly with its impact on the neurocognitive function and emotional well-being, continue to represent a serious danger and a very significant obstacle for achieving a close glycemic control. Moreover, following the subcutaneous administration of recombinant IGF-I, the onset of diabetes is only delayed from day 16 to day 18, but the final incidence of the disease beyond day 22 after the induction of diabetes is probably the same for untreated and treated groups (Bergerot et al., 1995).

In particular, Thivolet et al., in patent US 6342227, disclosed the administration of IGF-I as a method of delaying the clinical onset of diabetes. In their study, Thivolet et al. administered the recombinant IGF-I twice a day, probably because the half-life of IGF-I in the circulation is 9 min, although it can be further increased to 16 hours when it is bound to insulin-like growth factor binding proteins (IGFBP) (Janssen, 2008). In Thivolet's study, it was proposed that the protective action of IGF-I would arise due to the changes in the transit of compromised self-reactive T cells towards the islets of the recipient mouse. I.e. following the transfer of self-reactive T cells from diabetic donours, the treatment with IGF-I reduced the number of donour T cells in the spleen of the recipients.

In previous works at our laboratory, we demonstrated that the local expression of IGF-I in the pancreas of transgenic mice prevents infiltration of the islets and the death of β cells in a DT1 model, the RIP/hIFNβ mouse, which presents an increased susceptibility to developing diabetes (Casellas et al., 2006). Moreover, the expression of IGF-I in β cells of diabetic mice is capable of regenerating the endocrine pancreas in a transgenic model (George et al., 2002).

Taking into consideration the information comprised in the state of the art, the present invention focuses on the discovery of an alternative method capable of preventing or treating DT1, whilst resolving the problems associated with the methods known in the state of the art. In particular, the method developed in the present invention was capable of preventing or treating DT1 by means of a weekly hydrodynamic injection of a plasmid that allowed for the expression of IGF-I in the liver, whilst maintaining normal circulating levels of IGF-I. At no time was any abnormal increase in the levels of circulating free IGF-I observed. Moreover, the number of administrations necessary to prevent or treat DT1 in the present invention could be reduced, under the appropriate conditions, to a single injection. In a clinical environment, a single-dose regimen per treatment, or even a weekly application, would greatly increase the patients' quality of life. The strategy used in the present invention was capable of reducing the incidence of the disease by up to 80% and providing complete protection against DT1 after 10 administrations, even after withdrawing the treatment. Finally, the DNA-based treatment of the invention is more economical, easier to produce and safer than the administration of protein, and does not require refrigeration for short- or long-term storage.

### DESCRIPTION OF THE INVENTION

### BRIEF DESCRIPTION OF THE INVENTION

The invention relates to the specific expression, in both hepatocytes and non-parenchymal hepatic cells, of vectors which comprise at least one sequence that encodes IGF-I. Plasmids are particularly preferred vectors. The adverse hypoglycemic effect caused by the direct administration of IGF-I does not take place following the administration of gene therapy compositions that comprise DNA constructs which include a DNA sequence encoding IGF-I or a functional biological equivalent thereof, associated with at least one vector, particularly plasmids, adequate for the expression of IGF-I or the functional biological equivalent thereof in hepatic cells, probably due to the fact that IGF-I is not intensely synthesised by cells. In the present invention, a sequence of experiments were performed in order to assay the preventive effects of IGF-I against DT1 when administered in the form of a hydrodynamically injected plasmid encoding IGF-I.

One of the essential characteristics of the invention is that the gene therapy compositions and the administration thereof are designed for the expression of IGF-I in hepatocytes and non-parenchymal hepatic cells. This leads to physiological levels of circulating IGF-I, which prevents the undesireable effects that take place when the serum levels of IGF-I increase and cause undesireable effects in other target tissues: as an example, hypertrophia in the cardiac muscle tissue.

Therefore, a gene therapy focus was developed wherein the hepatic administration of a plasmid that expresses IGF-I leads to the prevention of the development of diabetic hyperglycemia in a mouse model of autoimmune diabetes. The interruption of the immune attack against pancreatic β cells in animals treated with IGF-I prevents the loss of β cell mass observed in diabetic mice and, consequently, the levels of circulating insulin are maintained. This protection against the disease could be mediated, at least partially, by an increase in the percentage of intrapancreatic regulatory T cells (T_{reg} cells), since an antibody-mediated depletion of this population makes it impossible to prevent the disease mediated by IGF-I.

More specifically, the present invention showed that the expression of IGF-I in the liver, by means of plasmids that have the CAG or CD68 promoters, was capable of mediating the protection against DT1. However, any promoter that may direct the expression of a transgene in parenchymal and non-parenchymal hepatic cells, such as CMV (van Til et al.), PGK (Uesugi et al.) or acetyl-LDL (Bradshaw et al.; Klein et al. 2007), could be potentially used to express transgenes in hepatic cells and, therefore, should be included within the scope of patent protection conferred to the present invention.

In a preferred embodiment of the invention, the plasmids effectively used to administer IGF-I were pCAG IGF-I WPRE (SEQ ID No.: 1) and pCD68 IGF-I (SEQ ID No.: 2).

Moreover, several vectors have proven to be hepatotropic and, therefore, could act as appropriate vectors to express IGF-I in hepatic cells. The present invention demonstrated that the hydrodynamic injection of a plasmid leads to the transfection of most classes of hepatic cells and, therefore, it is likely that the hydrodynamic injection of a viral vector would also do so. Consequently, the present invention suggests that the hydrodynamic injection of a viral vector that expresses IGF-I would be capable of expressing the transgene in hepatic cells and, therefore, protecting against DT1. Below we list examples of said vectors: adenoviral vectors, high-capacity adenoviral vectors (HC-Ad or gutless), AAV (Adeno-associated viruses), Poxviruses, Lentiviral vectors or viral vectors derived from the Herpes simplex virus.

When extrapolated to animals or human beings with greater body weights, the method of administration used in the experiments with mice, the so-called hydrodynamic injection, must be adapted. A description of a method similar to the hydrodynamic injection, adapted to pigs, is disclosed in Aliño et al. For purposes of the present invention, the method of administration of the gene therapy compositions to human beings is a local injection directly into the liver.

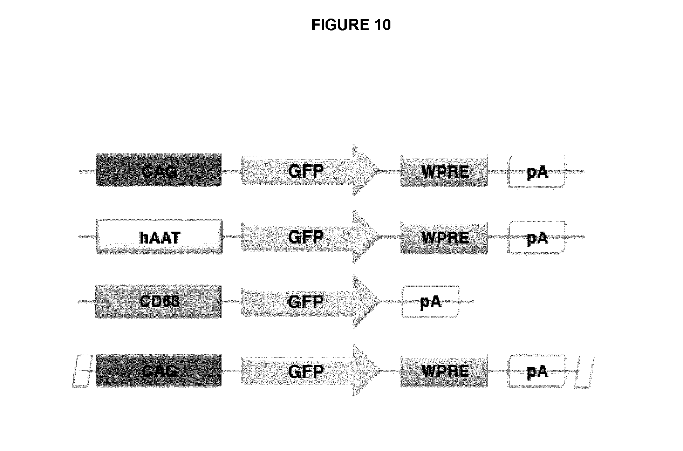

An example of a DNA construct comprised in a viral vector may be observed in Figure 10 of the present specification.

For purposes of the present invention, the functional biological equivalent expression of IGF-I may have a sequence that is identical to the sequence that is naturally found in IGF-I, but it may also have modified sequences that provide a function equivalent to that of a vector wherein said sequences are incorporated. In particular, said modifications may include the deletion of ALU repeated sequences of 3' UTR. This is only one example and it is non-limiting. The experimental confirmation of the invention claimed has been performed in experimental animal models, particularly in mice, and, therefore, the DNA used in the constructs for the gene therapy of DT1 encoded mouse IGF-I. However, the experimental results obtained may be extrapolated to human beings and, therefore, for purposes of the description of the present invention, the term IGF-I is indifferently applied to a polypeptide of mouse or human origin.

In another preferred embodiment, the sequence that encodes IGF-I is a synthetic IGF-I coding sequence. Said synthetic sequence has a nucleotide sequence different from a natural human IGF-I coding sequence. It is preferable for the sequence to make an optimal use of codons; preferably, at least 50%, 70% or 90% of the codons are optimised. Therefore, in preferred embodiments, the synthetic DNA sequence has a sequence identity of at least 80%, 90%, 95% or 99% with respect to the natural human IGF-I sequence. In a particular preferred embodiment, the synthetic DNA sequence has an identity of at least 95%, more preferably an identity of at least 99%, and, more preferably, an identity of 100% with respect to the natural IGF-I sequence.

Moreover, another embodiment of the preceding vector may contain a regulatory system to adjust the expression of the nucleic acid construct or cassette. The expression "regulatory system", as used in this document, refers to sequences incorporated into the preceding vectors acting in cis or in trans, which regulate any characteristic of the expression of the nucleic acid of interest, as well as gene products acting in trans which are co-expressed in the cell with the vector described above. Regulatory systems may be used for the positive or negative regulation of the expression from the normal levels of expression or existing levels of expression at the time of regulation. The system contributes to the temporal expression pattern of the polynucleotide.

A related aspect of the invention provides a formulation of a pharmaceutical composition for the administration and expression of an IGF-I gene in a cell, preferably a human IGF-I gene. The formulation includes a vector of the preceding aspect jointly with one or more pharmaceutically acceptable components which may, for example, act by stabilising the vector or increasing the transfection efficacy, but may also provide other functions. In a preferred embodiment, the formulation includes the vector in a solution containing polyvinylpyrrolidone (PVP) at between approximately 0.5% and 50%, preferably PVP at approximately 5%. Preferably, PVP has an average molecular weight of approximately 50,000 g/mol. Additional information is disclosed in document PCT US95/17038. However, another example of a formulation includes the vector with a cationic lipid (for example, as described in United States Patent 4.897.355, issued on 30 January 1990) and may also include a colipid, such as a neutral colipid.

Moreover, for purposes of the present invention, the expression "therapeutically effective dose" refers to the quantity of active ingredient that increases or decreases the activity with respect to the activity produced in the absence of the therapeutically effective dose. For purposes of the present description of the invention, the term "therapeutically" also includes the prevention of the onset of the disease. Therefore, for purposes of the present invention, the terms prevention or treatment refer to a preventive therapy or treatment which, on the one hand, may prevent the onset of the autoimmune disease and, on the other hand, may stop the progression of an autoimmune disease already ongoing or initiated, blocking the harmful effects of that autoimmune disease in the body.

For any compound, the therapeutically effective dose may be initially estimated in cell culture assays or animal models, habitually mice, rabbits, dogs or pigs. The animal model may also be used to determine the appropriate concentration interval and the administration route. Said information may be subsequently used to determine the useful doses and administration route in human beings.

The therapeutic efficacy and the toxicity, for example, the DE50 (the therapeutically effective dose in 50% of the population) and the DL50 (the lethal dose for 50% of the population) may be determined by conventional pharmaceutical processes in cell cultures or experimentation animals. The ratio between the doses with toxic effects and the therapeutic doses is the therapeutic index, and may be expressed as the ratio DL50/DE50.

Pharmaceutical compositions are preferred which present high therapeutic indices. The data obtained from cell culture assays and animal studies are used to formulate a dosage interval for human use. The dosage contained in said compositions is preferably within an interval of circulating concentrations that includes DE50, with little or no toxicity. The dosage varies within this interval depending on the dosage form used, the patient's sensitivity and the administration route.

The exact dosage will be determined by a skilled professional. The dosage and the administration are adjusted to provide sufficient levels of the active ingredient or to maintain the desired effect. The facts that must be taken into consideration include the severity of the pathology, the subject's general health, age, weight and gender, the diet, the time and frequency of administration, the combination or combinations of drugs, sensitivity reactions and the tolerance/response to the therapy. The normal dosage quantities may vary between 0.1 and 100,000 micrograms, to a total dose of approximately 1 g, depending on the administration route. The literature provides guidance regarding particular dosages and administration methods and is generally available to professionals skilled in the art. Specialists in the art will use different formulations for nucleotides than for proteins or the inhibitors thereof. Similarly, the administration of polynucleotides or polypeptides will be specific for particular cells, medical conditions, locations, etc.

In any of the embodiments described above, any of the pharmaceutical compositions of the invention may be administered combined with other appropriate therapeutic agents. The selection of the appropriate agents to be used in a combined therapy may be performed by a person skilled in the art in accordance with conventional pharmaceutical principles. The combination of therapeutic agents may act synergically to achieve the treatment or prevention of the various disorders described above. Using this approach, it may be possible to achieve therapeutic efficacy with lower dosages of each agent, thereby reducing the possibility of adverse secondary effects.

Any of the therapeutic methods described above may be applied to any subject that needs said therapy, including, for example, mammals such as dogs, cats, cows, horses, rabbits, monkeys and, more preferably, human beings.

All the patents and patent applications cited in this description are expressly included in this document as a reference as a whole. The preceding description describes the present invention in general. A more complete understanding may be obtained by referring to the following specific examples, which are provided for illustrative purposes only, and are not intended to limit the scope of the invention.

### DEPOSIT OF MICROORGANISMS IN ACCORDANCE WITH THE BUDAPEST TREATY

The plasmids mentioned above, pCAG IGF-I WPRE (SEQ ID No.: 1) and pCD68 IGF-I (SEQ ID No.: 2) were deposited at the Spanish Type Culture Collection (CECT); University of Valencia, Burjasot Campus, Burjasot (Valencia), Spain, with access numbers CECT7560 and CECT7561, respectively, the deposits at the Budapest Treaty Authorities having been made on 09-06-09.

### DESCRIPTION OF THE FIGURES

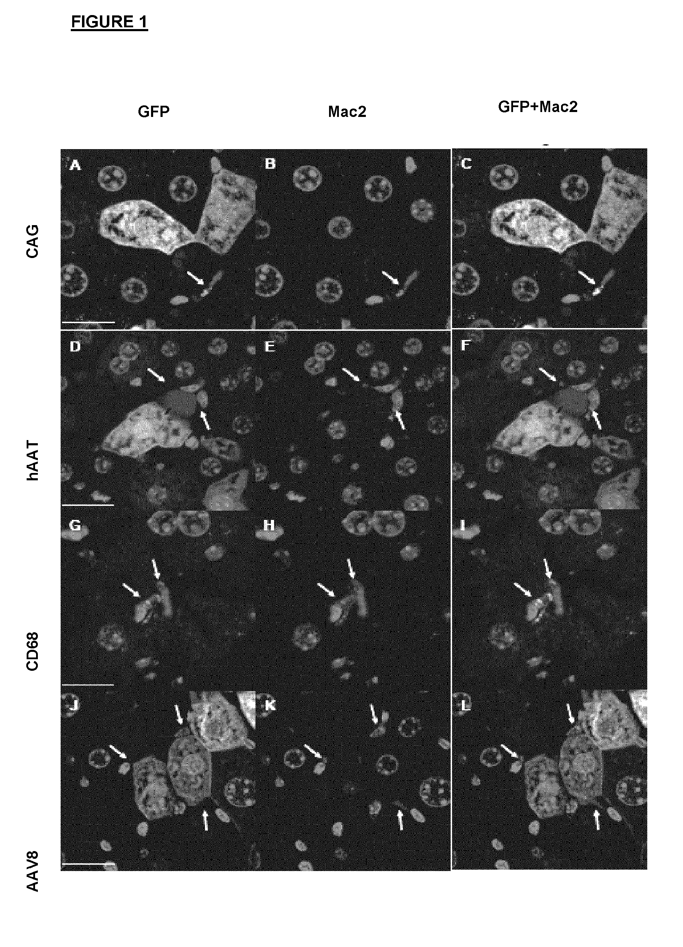

**Figure 1****.- Immunohistochemical analysis of the expression of GFP and Mac-2 in liver sections.** Twenty-four hours post-hydrodynamic injection of plasmids CAG, hAAT and CD68, which express GFP, and 1 week following the injection of vector AAV8 CAG GFP WPRE, the staining with specific anti-Mac-2 antibodies in mouse liver sections was examined. GFP⁺/Mac-2⁺ double positive cells were detected in mouse livers which had been injected with plasmids CAG-GFP and CD68-GFP (**A-C, G-I**), but not following the injection of plasmid hAAT-GFP (**D-F**). Vectors AAV8 lack Kupffer cell tropism, as revealed by the absence of GFP⁺/Mac-2⁺ cells in those animals which were injected with AAV8-CAG-GFP (**J-L**). The white arrows indicate the location of the Kupffer cells. Scale bars: 18 µm (A-C); 22 µm (D-F); 16 µm (G-I); 24 µm (J-L).

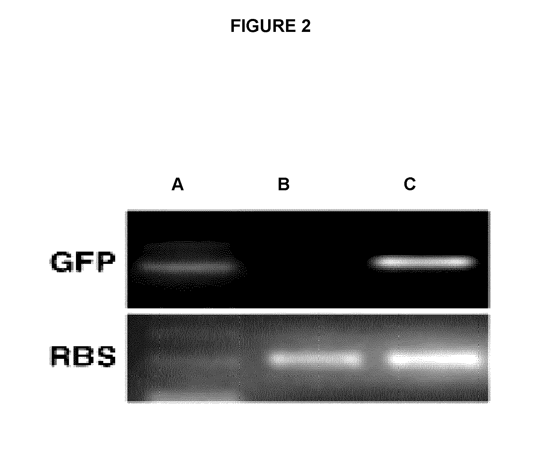

**Figure 2****.- Analysis of the expression of GFP by means of RT-PCR in separated CD11b⁺ cells**. The animals were injected with saline solution (B) or 20 µg of pCAG GFP WPRE (C). Twenty-four hours post-injection, hepatic CD11b⁺ cells were separated and the RNA was extracted. A single specific band for GFP was amplified in the group injected with plasmid DNA, which demonstrates that non-parenchymal cells expressed the GFP transgene. Lane (A) shows the molecular weight marker.

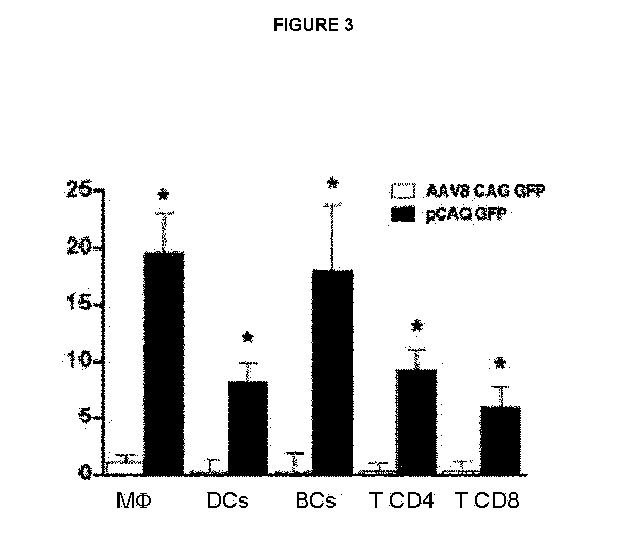

**Figure 3****.- Analysis by flow cytometry of the expression of GFP in non-parenchymal hepatic cells following the hydrodynamic injection of plasmid DNA.** Different hepatic cell populations were analysed by flow cytometry in those animals which were injected with pCAG and AAV8 CAG. The expression of GFP was detected in macrophages - CD11b⁺ Kupffer cells - (MΦ), CD11c⁺ dendritic cells (DCs), CD19⁺ B lymphocytes (BCs), and CD4⁺ and CD8⁺ T cells. In all the cell populations analysed, the percentage of GFP⁺ cells in animals which were injected with plasmid CAG was significantly greater than that obtained from animals injected with vector AAV8, which were practically negative for the presence of GFP. A representative experiment of 4 independent experiments is shown. The results are the mean ± standard error of the mean (SEM) of the relative number of GFP⁺ cells within each population. n = 4 mice per group. The Y-axis represents the % of GFP⁺ cells versus the total number of cells.

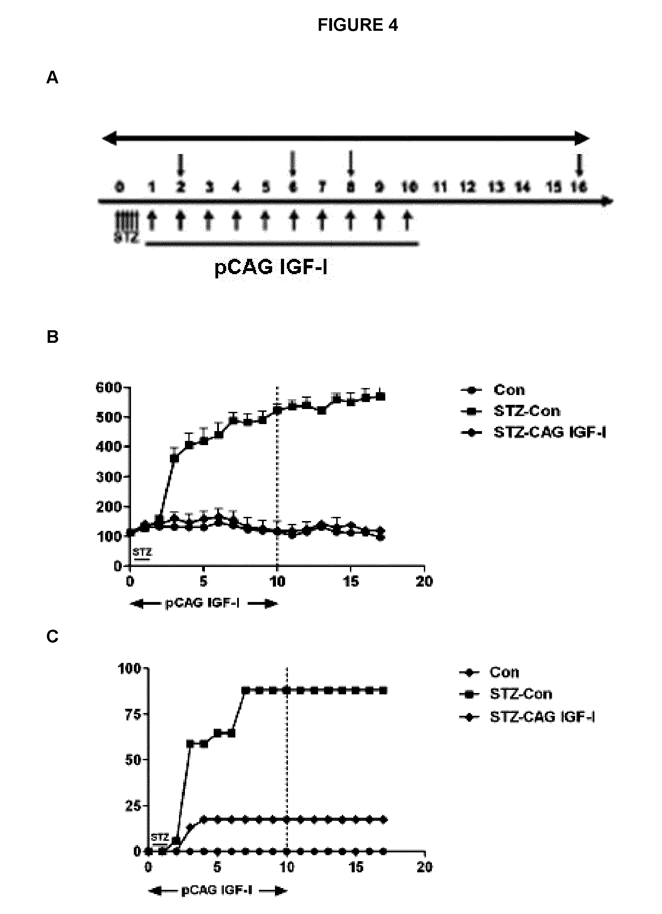

**Figure 4****.- Prevention of type 1 diabetes by the hepatic expression of IGF-I by means of the hydrodynamic injection of plasmid CAG-IGF-I. (A)** Tg IFNβ mice were administered 5 consecutive daily doses of STZ (25 mg/kg) at days 1-5. Tg IFNβ mice not treated with STZ were used as control animals (Con). At day 9, the STZ-Con mice were hydrodynamically injected with plasmid pVAX1, which lacks an expression cassette, or 5 µg of a plasmid that encodes IGF-I under the control of the CAG promoter (STZ-IGF-I). The mice were administered a weekly hydrodynamic injection for 10 weeks. Subsequently, the mice's glycemia was followed up for 6 additional weeks. The numbers indicate the number of weeks of duration of the treatment in the murine animal model. At week 2, an analysis of the replication of B cells, measured as the B cell mass, and an analysis of the presence of insulitis in all the study animals were performed. At week 6, a glucose tolerance test was performed. At week 8, the insulinaemia and the serum levels of IGF-I were measured in the study animals and, at week 16, the B cell mass and the insulitis that they presented was once again analysed in all the animals. The upper arrow indicates the follow-up of the glucose levels (glycemia) that the animals showed throughout the entire study. **(B)** Most of the mice treated with IGF-I did not develop hyperglycemia following the treatment with STZ. On the contrary, the animals treated with a void plasmid (STZ-Con) became highly hyperglycemic during the first few weeks following the administration of STZ. Those animals not treated with STZ (Con) maintained normoglycemia. The values are shown as the mean ± SEM. Only the normoglycemic animals in both STZ-IGF-I groups are shown. The end of the re-administration of pIGF-I is indicated by a dotted line. The Y-axis represents the concentration of glycemia expressed in mg/dl and the X-axis represents the time expressed in weeks. **(C)** In STZ-Con mice, the incidence of diabetes reached 90% (15/17) at week 8, whereas those animals treated with IGF-I showed a clear resistance to the development of hyperglycemia: 83% (19/23) of the animals were normoglycemic in the STZ-IGF-I group treated with 5 µg of plasmid DNA. None of the Con animals (not treated with STZ) became diabetic (0/19). The animals were considered to be diabetic when 2 consecutive blood glucose measurements were greater than 250 mg/dl. The results shown are representative of 3 independent experiments. The end of the re-administration of pIGF-I is indicated by a dotted line. The Y-axis represents the % of diabetic animals and the X-axis represents the time expressed in weeks.

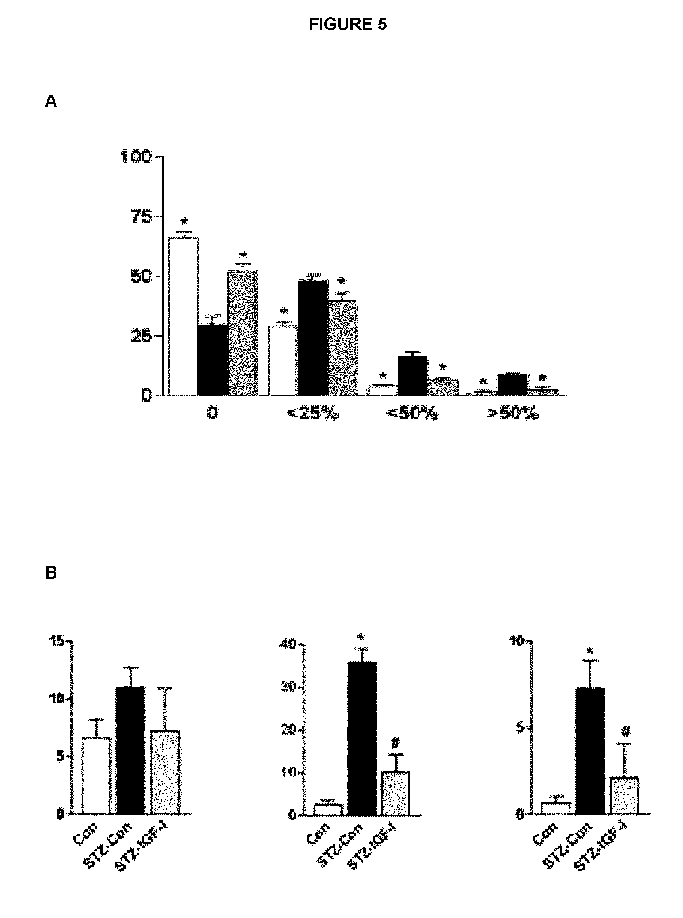

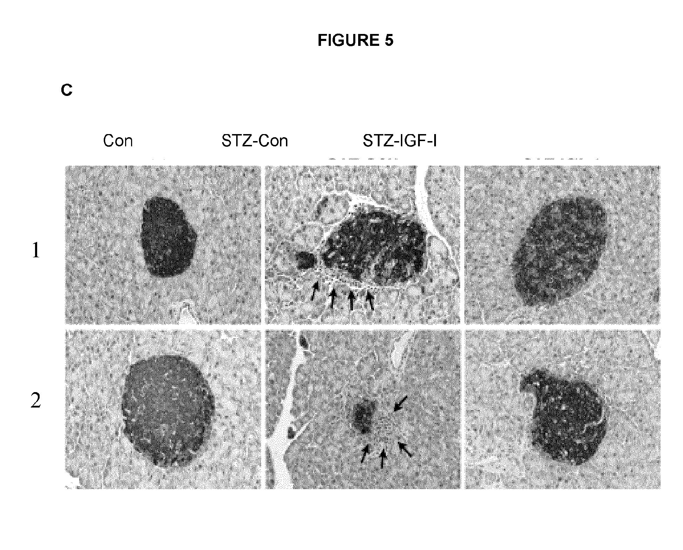

**Figure 5****.- Analysis of the lymphocyte infiltration of pancreatic islets following the treatment with IGF-1. (A)** Quantification of the pancreatic islets that presented lymphocyte infiltration. The levels of insulitis were determined in control animals (white bars) and animals treated with STZ (black bars) and with STZ-IGF-I (grey bars) after 15 days of treatment. The Y-axis represents the percentage of lymphocyte infiltration in the pancreatic islets of samples from said animals. The degree of infiltration was measured using the following ranking: not infiltrated (0); peri-insulitis (< 25%); moderate insulitis (< 50%); severe insulitis (> 50%). *p < 0.05 vs STZ-control. **(B)** The insulitis was quantified by means of flow cytometry 15 days after the treatment with STZ. The percentage of infiltrating CD4⁺ and CD8⁺ T cells was lower in the animals treated with pCAG IGF-I. The results show the mean ± SEM of 3 animals per group. *p < 0.05 vs Con. # p<0.05 vs STZ-con. The Y-axes of each of the graphs show the percentages of CD11b⁺, CD4⁺ and CD8⁺ cells, respectively. **(C)** Detection by immunohistochemistry of the insulin present in pancreatic tissue sections of control animals not treated with STZ (Con), control animals treated with STZ (STZ-Con) and animals treated with STZ-IGF-I, at two weeks (1) and at 4 months (2) from the beginning of the treatment. Magnification 100x.

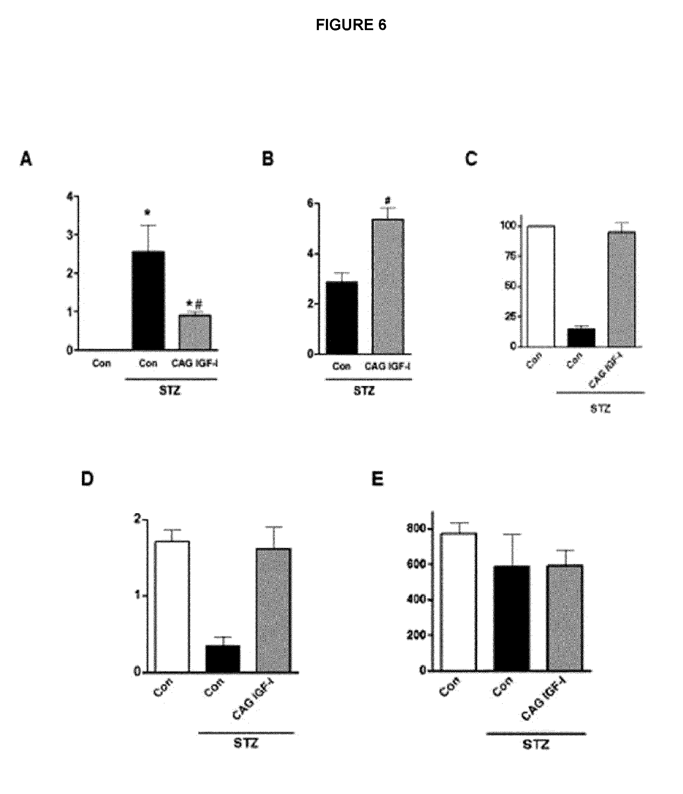

**Figure 6****.- Analysis of the apoptosis and replication of** β **cells, the** β **cell mass and the circulating levels of insulin and IGF-I. (A)** Two weeks after the injection of STZ, the percentage of apoptotic β cells was determined in mice not treated with STZ (Con), in mice treated with STZ (STZ-Con) and in mice treated with STZ which had been administered a plasmid encoding IGF-I (STZ CAG IGF-I). The results are the mean ± SEM of four mice per group. (* p < 0.05 versus Con. # p < 0.05 versus STZ-Con). The Y-axis shows the percentage of apoptotic β cells. **(B)** An analysis of the replication of β cells was performed in Con, STZ-Con and STZ CAG IGF-I mice 2 weeks after the treatment with STZ. Three pancreatic sections per animal were immunostained against insulin and Ki67, and the frequency of Ki67-positive β cell nuclei was determined. The results are the mean ± SEM of four mice per group. (# p < 0.05 versus STZ-Con). The Y-axis represents the percentage of Ki67⁺ β cells. **(C)** The β cell mass was measured in mice not treated with STZ (Con) and in mice treated with STZ which were injected with void plasmid (STZ-Con) and treated with IGF-I (STZ IGF-I) four months after the administration of STZ. Nine pancreatic sections of each individual mouse were analysed. The results are the mean ± SEM of 4 animals per group. (* p < 0.05 versus Con). The Y-axis represents the β cell mass as a percentage with respect to the control animals. **(D)** The insulinaemia was measured by means of an enzyme-linked immunosorbent assay (ELISA) 7 weeks after the first administration of IGF-I. The normoglycemic animals treated with IGF-I maintained normal insulin values, whereas the STZ-Con animals had very low levels of the hormone. The results are expressed as the mean ± SEM of 4 animals per group. (* p < 0.05 versus Con). The Y-axis represents the insulin concentration expressed as ng/ml. **(E)** The serum levels of IGF-I at 7 weeks after the first administration of IGF-I, measured by ELISA, did not show any differences between the groups. The results are the mean ± SEM of 4 animals per group. The Y-axis represents the serum levels of IGF-I expressed as ng/ml.

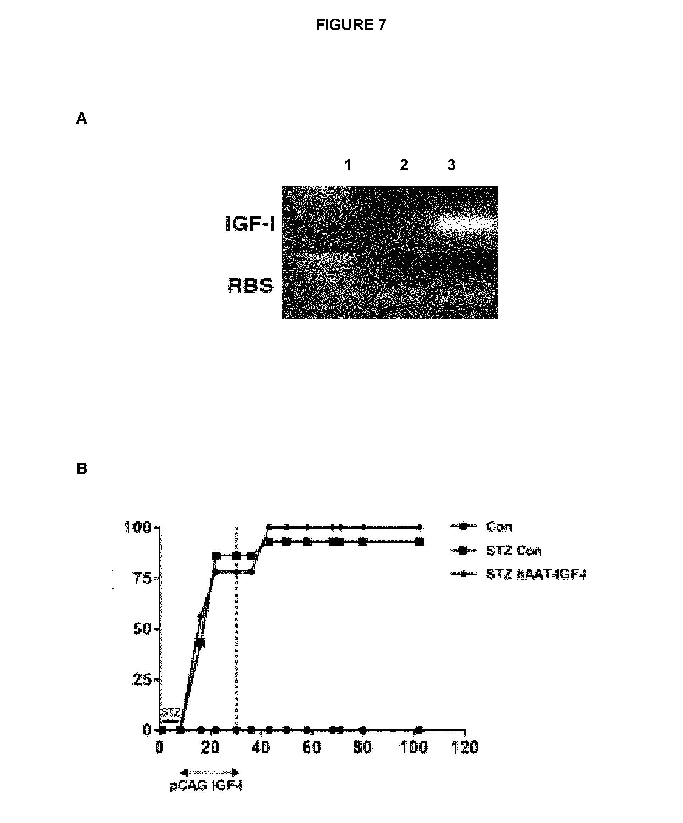

**Figure 7****.- Cumulative incidence of diabetes following the HTV injection of a plasmid that expresses IGF-I under the control of a hepatocyte-specific promoter (phAAT-IGF-I). (A)** The total RNA was extracted from the liver 24 h after the hydrodynamic injection of 5 µg of phAAT IGF-I and retrotranscribed to cDNA. Specific primers were used to detect the expression of IGF-I from the plasmid following the hydrodynamic injection by means of RT-PCR. Lane 1 shows the molecular weight marker. Lane 2 shows the results in control animals injected with saline solution and lane 3 shows the results of the animals injected with phAAt-IGF-I. **(B)** The cumulative incidence of diabetes reached approximately 90% (13 of 14 animals) in STZ-Con mice at 2 weeks post-treatment with STZ. The animals that expressed IGF-I under the control of a hepatocyte-specific promoter did not show prevention of diabetes (12/12). The animals were considered to be diabetic when 2 consecutive blood glucose measurements were greater than 250 mg/dl. A representative experiment of 3 separate experiments is shown. The suppression of the treatment with IGF-I is indicated by a dotted line. The X-axis represents the time expressed in days and the Y-axis represents the cumulative incidence percentage of diabetes.

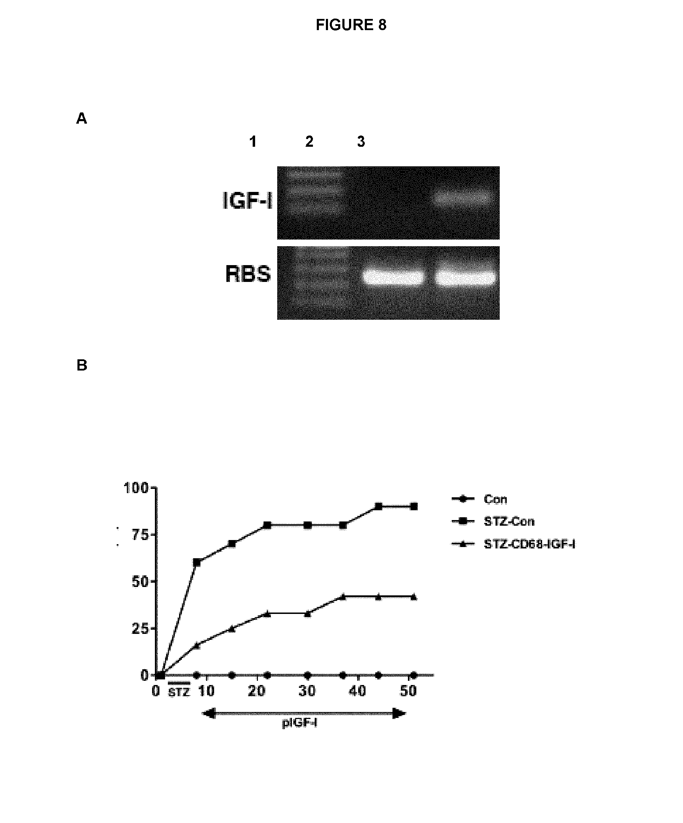

**Figure 8****.- Cumulative incidence of diabetes following the hydrodynamic injection of a plasmid that expresses IGF-I under the control of a myeloid-cell-specific promoter (pCD68-IGF-I). (A)** The total RNA was extracted from the liver 24 h after the hydrodynamic injection of 5 µg of pCD68 IGF-I and retrotranscribed to cDNA. Specific primers were used to detect the expression of IGF-I from the plasmid following the hydrodynamic injection by means of RT-PCR. Lane 1 shows the molecular weight marker. Lane 2 shows the results in the control animals injected with saline solution and lane 3 shows the results of the animals injected with pCD68 IGF-I. **(B)** The cumulative incidence of diabetes reached 90% at week 6 in STZ-Con mice (9 of 10 mice). The animals that expressed IGF-I under the control of the CD68 promoter were protected against the disease, since only 40% (5/12) of them became diabetic at week 6 post-STZ. None of the mice that were not treated with STZ developed hyperglycemia. The animals were considered to be diabetic when 2 consecutive blood glucose measurements were greater than 250 mg/dl. A representative experiment of 2 separate experiments is shown. The X-axis represents the time expressed in days and the Y-axis represents the cumulative incidence percentage of diabetes.

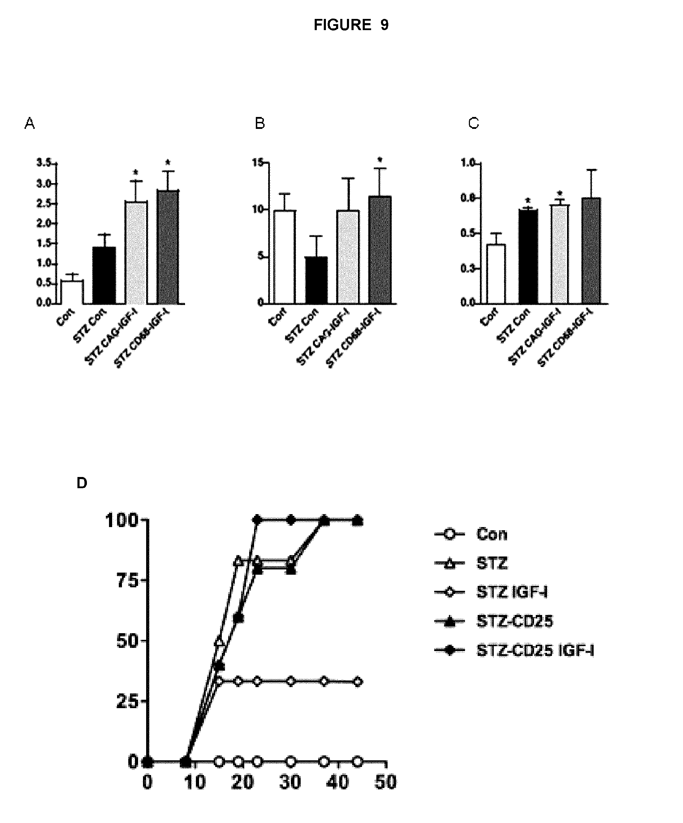

**Figure 9****.- Percentage of regulatory T cells (T_{reg}) following the treatment with pIGF-I and cumulative incidence of diabetes following the depletion of T_{reg} cells.** One month after the induction of diabetes, intrahepatic **(A),** intrapancreatic **(B)** and intrasplenic **(C)** lymphocytes were isolated and analyzed by flow cytometry, following the staining with specific antibodies against CD4, CD25 and GITR. The percentage of T_{reg} in the liver and the pancreas of mice treated with IGF-I had increased, whereas the intrasplenic T_{reg} remained unchanged. The values are shown as the mean ± SEM. n = 4 animals per group. The Y-axis of graphs (A), (B) and (C) represents the percentage of CD25⁺GITR⁺ cells versus CD4⁺ cells. **(D)** The depletion of CD4⁺ CD25⁺ T_{reg} cells using anti-CD25 monoclonal antibody resulted in the loss of the protective effects of IGF-I, since 100% of the animals treated with pIGF-I were diabetic at day 30 post-STZ. Over 80% of the animals which had been administered rabbit serum (NRS) as a control, as well as a void plasmid, also developed the disease. Only one-third of the pIGF-I + NRS animals became diabetic. n = 5-6 mice per group. The X-axis represents the time expressed in days and the Y-axis represents the cumulative incidence percentage of diabetes.
**Figure 10****.- Schematic representation of the constructs that encode the expression of GFP.** Plasmids pCAG, phAAT and pCD68 used for the studies of the expression of indicator genes are shown from top to bottom. Below these, vector AAV8-CAG is shown. The CAG promoter is a hybrid promoter composed of the CMV early/intermediate enhancer, the chicken β-actin promoter and the first intron of the human β-globin gene. It is ubiquitously expressed. The hAAT promoter is a hybrid promoter composed of the hepatocyte control region (HCR) enhancer of apolipoprotein E and the human α-antitrypsin promoter. Its expression is limited to hepatocytes. The CD68 protein is a classic macrophage marker. Its expression should be primarily limited to Kupffer cells. The vectors are not to scale.

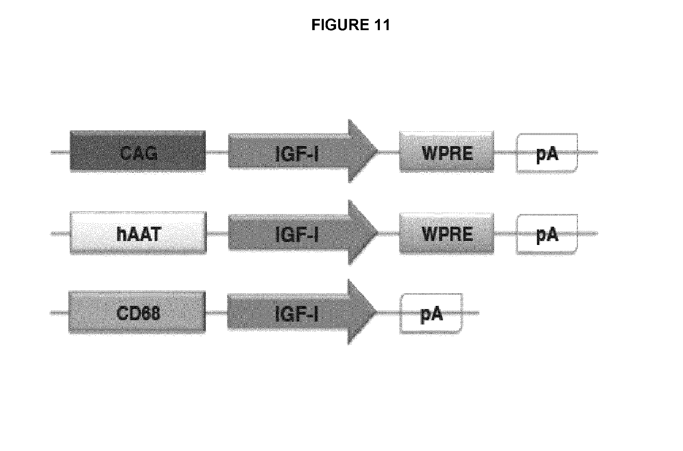

**Figure 11****.- Schematic representation of the constructs that express IGF-I.** Plasmids pCAG, phAAT and pCD68 used for the studies on the preventive actions of IGF-I against type 1 diabetes are shown from top to bottom. The vectors are not to scale.

### DETAILED DESCRIPTION OF THE INVENTION

As described above, a gene therapy focus was developed in the present invention wherein the hepatic administration of a plasmid that expresses IGF-I leads to the prevention of the development of diabetic hyperglycemia in an autoimmune diabetes mouse model. The interruption of the immune attack against pancreatic β cells in the animals treated with IGF-I prevents the loss of β cell mass observed in diabetic mice and, therefore, the levels of circulating insulin are maintained.

In the present invention, the synthesis of IGF-I is performed from vectors that comprise an adequate promoter for expression in hepatic cells. The preferred vectors are plasmids that comprise CAG or CD68 as adequate promoters, resulting in a constant supply of IGF-I. Moreover, in the present invention, the preventive use of IGF-I is achieved by administering a weekly hydrodynamic injection of a solution carrying a plasmid that contains the necessary genetic information for the cells to produce IGF-I. In the present invention, a treatment was established which consisted of one weekly administration of DNA, by contrast with the daily injections used by others in the state of the art. The present invention demonstrated that the animals achieved complete protection against DT1 after 10 administrations of a plasmid that encodes IGF-I, even after the treatment was withdrawn. These points acquire special interest when we take into consideration that the prevalence of diabetes is rapidly increasing in developing countries, where repeated treatments with insulin are usually too costly and inaccessible for many patients, leading to a very heavy sanitary, social and economic burden.

One of the most significant aspects of the present invention is that the therapy with IGF-I reduced the incidence of diabetes: 90% of the mice developed diabetes in the STZ-Control group, whereas 80% of the animals in the group treated with IGF-I did not develop the disease. However, a small percentage of the animals treated with IGF-I became diabetic. Therefore, the treatment with IGF-I does not delay the onset of the disease, but decreases the incidence of DT1. Significantly, after a period of weekly treatments that has been estimated at 10 weeks, the animals are completely protected against the disease and do not develop diabetes when the treatment is withdrawn.

The invention, which uses IGF-I, leads to an intervention in the immune system the final result whereof is the interruption of the autoimmune attack against the pancreatic islets by the infiltration of CD4⁺ and CD8⁺ T cells, probably through the involvement of regulatory T cells (T_{reg}). This leads to protection against autoimmune diabetes. Moreover, the results of the experiments demonstrate that IGF-I does not prevent the onset of diabetes when the regulatory T cells have been reduced by means of a specific antibody. These observations relate the protective effect achieved by IGF-I with the presence of regulatory T cells. T_{reg} cells are a specialised sub-population of T cells that act by suppressing the activation of the immune system and, therefore, maintain the homeostasis of the immune system and tolerance to autoantigens. Recent advances in the molecular characterisation of this cell population have firmly established its existence and its critical role in the immune system of vertebrates. Interest in regulatory T cells has increased due to signs in experimental mouse models that demonstrate that the immunosuppressive potential of these cells may be therapeutically utilised to treat autoimmune diseases.

Taking into consideration all these results, it is suggested that this focus would be advantageous for all those autoimmune diseases wherein an attack of the immune cells (such as CD4⁺ and CD8⁺ T cells) against the target organ is involved in the development of the disorder.

Examples include type 1 diabetes (Kukreja et al., 2002; Lindley et al., 2005), multiple sclerosis (Viglietta et al., 2004), autoimmune vasculitis (Boyer et al., 2004), rheumatoid arthritis (RA) (Cao et al., 2003) and Langerhans cell histiocytosis (LCH) (Senechal et al., 2007), graft-versus-host disease (Cohen and Boyer, 2006), autoimmune proliferative syndrome type II (Kriegel et al., 2004), atherosclerosis (George, 2008), Crohn's disease (Ricciardelli et al., 2008), myasthenia gravis (Meriggioli et al., 2008), psoriasis (Sugiyama et al., 2005), Graves' disease (Pan et al., 2009), myositis (Banica et al., 2009), ulcerative colitis (Holmen et al., 2006) and lupus erythematosus (Kuhn et al., 2009; Valencia et al., 2007), ankylosing spondylitis (Forger et al., 2009), uveitis (Chen et al., 2008), primary biliary cirrhosis (Lan et al., 2006) and autoimmune hepatitis (Vergani and Mieli-Vergana, 2008).

Examples in animal models include experimental autoimmune encephalomyelitis (EAE) (Luth et al., 2008), experimental autoimmune gastritis (EAG) (McHugh, 2005), lupus-erythematosus-like disease (Kuhn et al., 2009), type 1 diabetes (Chen et al., 2005; Setoguchi et al., 2005), ulcerative colitis (Denning et al., 2005), experimental myasthenia gravis (Sheng et al., 2008), experimental autoimmune myositis (Allenbach et al., 2009) and uveitis (Matta et al., 2008).

In sum, the present invention shows a gene therapy focus wherein the hepatic administration of a vector that expresses IGF-I leads to protection against the development of diabetic hyperglycemia or other autoimmune diseases in both human beings and autoimmune diabetes mouse models. The interruption of the immune attack against pancreatic β cells in the animals treated with IGF-I prevents the loss of β cell mass observed in diabetic mice and, therefore, the levels of circulating insulin are maintained. This protection against the disease is considered to be mediated, at least in part, by an increase in the percentage of both intrahepatic and intrapancreatic T_{reg} cells, since an antibody-mediated depletion of this population hinders the prevention of the disease induced by IGF-I.

Therefore, the first embodiment of the present invention relates to a DNA construct that comprises a nucleotide sequence that encodes IGF-I and an adequate promoter to express said IGF-I in hepatic cells. Preferred promoters such as CAG or CD68 are selected. In a preferred embodiment of the invention, the DNA construct consists of plasmid pCAG IGF-I WPRE, characterised by SEQ ID No.: 1, or plasmid pCD68 IGF-I, characterised by SEQ ID No.: 2.

The second embodiment of the invention relates to the use of said DNA construct for the manufacturing of active pharmaceutical compositions for the treatment of DT1 or any other autoimmune disease, particularly, without being limited thereto, autoimmune diseases caused by the dysfunction or de-regulation of T_{reg} cells.

The third embodiment of the invention relates to a pharmaceutical composition that comprises the DNA construct mentioned above and a pharmaceutically acceptable vehicle.

The excipients, vehicle substances and auxiliary substances must be pharmaceutically and pharmacologically tolerable, such that they may be combined with other compounds of the formulation or preparation and do not have any adverse effects on the organism treated. The pharmaceutical compositions or formulations include those that are suitable for oral or parenteral administration (including subcutaneous, intradermal, intramuscular and intravenous), although the best administration route depends on the patient's condition. The formulations may be in the form of individual doses. The formulations are prepared in accordance with methods known in the field of pharmacology. The quantities of active substances to be administered may vary depending on the particularities of the therapy.

The fourth embodiment of the invention relates to a method for the treatment or prevention of DT1 or any other autoimmune disease, which comprises the administration of the aforementioned composition to the patient. The method is **characterised in that** the composition is administered to the patient by hydrodynamic injection once a week, or even once per treatment. The method was capable of reducing the incidence of DT1 by up to 20% and providing complete protection against DT1 following 10 administrations.

### EXAMPLES

The following examples are intended to illustrate the invention, without limiting the scope thereof, and to demonstrate some important phenomena. In the first place, non-parenchymal hepatic cells (NPC) in the liver and parenchymal cells were capable of expressing the transgene following the hydrodynamic tail vein injection process. In the second place, the hydrodynamic injection of a plasmid that encodes IGF-I leads to the prevention of DT1 in an autoimmune diabetes mouse model. In the third place, the administration of IGF-I in the circulation of muscle or hepatic tissues is not sufficient to prevent autoimmune diabetes in the RIP/hIFNβ model.

### Example 1. Transgenic mice

Transgenic CD1 mice (N10 generation) that express human interferon beta (IFN-β) in β cells were used. All the experiments were performed in male transgenic mice. The transgenic mice were administered an intraperitoneal injection of STZ (25 mg/kg body weight), dissolved in 0.1 mol/l citrate buffer (pH 4.5) immediately prior to the administration, on 5 consecutive days. The mice were fed with a conventional diet *ad limitum* and kept in a 12-h light-darkness cycle (the lights were turned on at 8:00 am). The animal care and experimentation processes were approved by the Ethics Committee on Animal and Human Experimentation of the Universitat Autònoma of Barcelona.

### Example 2. Immunohistochemistry and histopathology

The tissues were fixed for 12-24 h in formalin, imbibed in paraffin and sectioned. For the immunohistochemical detection, the sections were incubated at 4°C overnight with specific antibodies for each protein, washed with PBS 3 x 5 min and incubated with the corresponding secondary antibodies. For the detection of fluorescence, the sections were incubated with fluorochrome-conjugated secondary antibody for 1 h at room temperature and the nuclei were counterstained with Topro or Hoechst. The bright-field detections were developed using the ABC complex (Vector Laboratories, United Kingdom), which uses 3'3'-diaminobenzidine (DAB) as the chromogenic substrate. The sections were counterstained in hematoxylin from Mayer. For the immunohistochemical detection of insulin, glucagon, somatostatin and pancreatic polypeptide proteins, pancreas were fixed for 12-24 h in formalin, imbibed in paraffin and sectioned. The sections were subsequently incubated at 4°C overnight with guinea pig anti-porcine insulin antibody (Sigma Chemical, St. Louis, MO) at a 1:100 dilution, rabbit anti-human glucagon antibody (Signet Labs, Dedham, MA) at a 1:4,500 dilution, rabbit anti-human somatostatin antibody (Serotec, Oxford, United Kingdom) at a 1:750 dilution or rabbit anti-human pancreatic polypeptide antibody (ICN Biomedicals) at a 1:2,000 dilution. As secondary antibodies, peroxidase-coupled rabbit anti-guinea-pig IgG (Dako, Glostrup, Denmark) or biotinylated goat anti-rabbit antibody (Pierce, Rockford, IL) and ABC complex (Vector Laboratories, Burlingame, CA) were used.

The incidence and severity of insulitis was analysed in 3 paraffin sections per pancreas separated by 100-150 µm, stained against insulin using the aforementioned antibody (section 1.3) and counterstained with hematoxylin. The degree of infiltration of mononuclear cells (percentage of insulitis) was measured using the following classifications: not infiltrated, peri-insulitis (mononuclear cells surrounding islets and ducts but without infiltration of the islet architecture), moderate insulitis (mononuclear cells infiltrating < 50% of the islet architecture) and severe insulitis (> 50% of the islet tissue infiltrated by lymphocytes and/or loss of the islet architecture).

### Example 3. Morphometric analysis and determination of the β cell mass

Pancreas were obtained from transgenic mice and the immunohistochemical detection of insulin was performed in three sections (2-3 µm), separated by 200 µm. The surface area (in square micrometres) of each islet and the surface area of each section were determined using a Nikon Eclipse E800 microscope (Nikon, Tokyo, Japan) connected to a video camera with a colour monitor and an image analyser (analySIS 3.0; Soft Imaging System, Lakewood, CO). The percentage of β cell surface area in the pancreas was calculated by dividing the surface area of all the insulin-positive cells in a section by the total surface area of this section and multiplying the result by 100. The β cell mass was calculated by multiplying the weight of the pancreas by the percentage of β cell surface area.

The replication of β cells was measured by the immunohistochemical analysis of the Ki67 marker. Ki67 is expressed at low levels during the initial G1 phase and is accumulated during the following S, G2 and M phases of the cell cycle; therefore, it is habitually used as a replication marker.

The samples were deparaffinised and treated with Proteinase K (10 µg/ml) with Tris/HCl, pH 7.6, for approximately 10 min at 37°C, and permeabilised with Triton X (0.1%) in PBS for 2 min. Finally, a double staining was performed using anti-insulin antibody (1:100) and anti-Ki67 antibody (1:200) in order to detect the replication of β cells. These primary antibodies and their secondary antibodies, jointly with the labelled streptavidin and Hoechst (used to counterstain the nuclei and discard false positive cells), are described in Section 3 of this chapter. At least 1000 nuclei of islet β cells per pancreas were counted and four animals per group were examined.

Apoptotic cells in deparaffinised pancreatic sections were identified using transferase-mediated dUTP nick-end labelling (TUNEL) (*in situ* cell death detection kit; Roche Molecular Biochemicals). No β cells were stained with an antibody cocktail (anti-glucagon, anti-somatostatin and anti-pancreatic polypeptide). The nuclei were counterstained with Hoechst (Sigma Chemical). The β cell was considered to be apoptotic when it was positive for TUNEL and negative for staining with an antibody cocktail. In these sections, the layer of non-β cells showed a red cytosolic staining, whereas the TUNEL-positive cells had green nuclei. At least 1000 nuclei of islet β cells per pancreas were counted and 4 animals per group were examined.

### Example 4. Analysis by Flow Cytometry

For the analysis of non-parenchymal hepatic cells and splenic cells by flow cytometry, the livers and spleens obtained from control mice were mechanically broken up and treated using the flat portion of a 10-ml syringe piston. The homogenate was subsequently run through 30-µm-pore pre-separation filters (Milteny Biotec), to obtain a suspension of a single cell. The cell suspension was gently coated on a Histopaque-1.083 (Sigma-Aldrich) and centrifuged at 800 g for 20 min. The non-parenchymal cells were collected from the interface. Intrapancreatic lymphocytes were obtained from control mice and treated following enzymatic digestion with collagenase, mechanical breakdown and discontinuous density gradient purification, using a Histopaque-1.083 (Sigma-Aldrich).

The following conjugated mAbs were used for the cell staining: PE-labelled and PE-Cy7-labelled anti-CD11b (M1/70), PE-labelled anti-CD4 (GK 1.5), anti-CD8 (53-6.7), anti-CD19 (6D5), FITC-conjugated anti-F4/80 (BM8), PE-conjugated anti-CD49 (BioLegend), FITC-conjugated anti-CD25, PE-CY7-conjugated anti-GITR (YGITR765), PE-conjugated anti-CD11 c (HL3) (BD Pharmingen). Antibodies of the same isotype served as controls. The data were obtained in an FC 500 MPL dual-laser cytometer (Beckman Coulter).

### Example 5. PCR

A conventional PCR was used to detect the expression of GFP in Kupffer cells separated by FACS and that of IGF-I from plasmid in the total liver, using GoTaq® DNA Polymerase (Promega, United States). The following primers were used: SEQ ID No.: 3 (GFP Fw primer), SEQ ID No.: 4 (GFP Rv primer), SEQ ID No.: 5 (IGF-I Fw primer) and SEQ ID No.: 6 (IGF-I Rv primer).

### Example 6. Hydrodynamic tail vein injection

A hydrodynamic tail vein injection was performed, as described in Liu et al., 1999. Plasmid DNA was diluted in saline solution in a volume (ml) equal to ∼10% of the mean body weight of the animals (grams), and manually injected into the lateral tail vein in less than 5 seconds. Prior to the injection, the animals were placed under a 250-W infrared heat lamp for a few minutes in order to dilate the blood vessels and facilite observation and simpler access to the tail vein. A plastic immobilisation device (Harvard Apparatus) was used to hold the animal for the injection. No anaesthesia was used, since it is not necessary, provided that an appropriate immobilisation device is used. 9.525-mm (3/8 inches) 26G-calibre hypodermic needles (BD) were used, the largest possible needle calibre that perfectly fits the access vein in order to inject the animals.

### Example 7. Statistical analyses

Statistical analyses were performed using the GraphPad Prism 4.0 computer programme (GraphPad Software, United States). All the data are shown as the mean ± SEM, except as otherwise indicated. When the data followed a normal distribution, a t-test was used for independent samples in order to compare 2 groups. The statistical significance was adjusted to P < 0.05.

### Example 8. Analysis of the hepatic transfection following the hydrodynamic process

The hydrodynamic tail vein injection of plasmid DNA is a potent tool for the transfer of genes into the mouse liver. It consists of a tail vein injection of saline solution in 5 seconds which contains plasmid DNA, equivalent to 8%-10% of the body weight. Despite the large percentage of hepatic cells that may be transfected using this method, all the data published thus far described that the transfection of the plasmid supplied was limited to hepatocytes (Budker et al, 2006). However, in their expression analyses, Budker et al. did not use specific antibodies against non-parenchymal cells to unequivocally identify the non-parenchymal cells transduced as such. In order to fully characterise the repertoire of transduced hepatic cells, making use of sensitive techniques, such as a specific immunohistochemical analysis, we subsequently analysed whether other types of hepatic cells could also be transfected following the hydrodynamic injection process. Mice were injected using a number of different constructs that express GFP under the control of ubiquitous or cell-specific promoters, as summarised in **Table 1**. The following constructs were used: a) a plasmid that encodes GFP under the control of a ubiquitous promoter (pCAG GFP WPRE); b) a plasmid that encodes GFP under the control of a hepatocyte-specific promoter (phAAT GFP WPRE); c) a plasmid that encodes GFP under the control of a myeloid-cell-specific promoter (pCD68 GFP), and d) an AAV8 vector that encodes GFP under the control of a ubiquitous promoter (AAV8 CAG GFP WPRE). The mice were sacrificed 24 hours post-injection of the plasmid or 5 days after the injection of AAV8. The livers were extirpated and hepatic sections were stained in order to detect the presence of GFP in hepatocytes or Kupffer cells (Mac2⁺ cells). **Figure 1** shows that, in addition to hepatocytes, some Mac-2⁺ Kupffer cells were also GFP⁺ following the HTV injection of pCAG GFP WPRE (A-C), which suggests the transfection of Kupffer cells. As expected, following the injection of phAAT GFP WPRE, many GFP⁺ hepatocytes were observed, but no GFP⁺ Kupffer cells were detected. Following the administration of pCD68 GFP, no GFP⁺ hepatocytes were observed, but many GFP⁺ Kupffer cells were detected (G-I). Finally, following the injection of AAV8 GFP WPRE, the Kupffer cells remained GFP-negative, whereas a significant percentage of the hepatocytes were GFP-positive (J-L).

**Table 1**

| **Name** | **Promoter** | **Gene of interest** | **WPRE** | **Poly A** | **Expected expression** |
|---|---|---|---|---|---|
| p C A G G F P WPRE | CAG | GFP | yes | SV40 | Ubiquitous |
| phAAT GFP WPRE | HCR/hAAT | GFP | yes | SV40 | Hepatocytes |
| pCD68 GFP | CD68 | GFP | no | SV40 | Kupffer cells |
| AAV CAG GFP WPRE (AAV vector) | CAG | GFP | yes | SV40 | Hepatocytes |

The maps of each corresponding plasmid are shown in Figures 10-11 for each of the constructs represented in Table 1.

### Example 9. Analysis of the expression of GFP in Kupffer cells separated by FACS

Since Kupffer cells (KC) eliminate large, insoluble waste fragments by phagocytosis, part of the GFP positivity detected in KCs after the injection of plasmid pCAG GFP WPRE could be the result of the phagocytosis of GFP from hepatocytes. However, following the administration of vector AAV8-CAG-GFP, no GFP⁺ Kupffer cells were detected (Figure 1 J-L). However, in order to discard this possibility, CD11b⁺ cells from liver homogenates enriched in non-parenchymal cells were separated by FACS following the hydrodynamic injection of plasmid pCAG GFP WPRE. Subsequently, an analysis by RT-PCR was performed in these separated cells in order to detect the expression of the GFP transcript 24 hours after the hydrodynamic injection. As shown in **Figure 2**, the CD11b⁺ cells expressed the transgene following the hydrodynamic injection process. This is the first proof described that the hydrodynamic injection of a plasmid may effectively transfect non-parenchymal hepatic cells.

### Example 10. Analysis by flow cytometry of non-parenchymal hepatic cells transduced following a hydrodynamic injection of plasmid

In addition to liver sinusoidal endothelial cells (LSEC) and Kupffer cells, hepatic sinusoids also house resident dendritic cells and a heterogeneous population of intrahepatic lymphocytes, which contains the subpopulation of pit cells which represent liver-specific natural killer cells (NK). In order to fully characterise the repertoire of hepatic NPCs that could be transfected following the hydrodynamic injection process, an analysis by flow cytometry was performed. Mice were hydrodynamically injected with 20 µg of pCAG-GFP WPRE, sacrificed 24 hours later and non-parenchymal hepatic cells were isolated. A set of animals were also injected with 2.5 x 10¹¹ viral genomes/animal of AAV8 CAG GFP WPRE and the livers were extirpated at 7 days post-injection. The non-parenchymal cells were stained with specific antibodies for the different types of hepatic cells: CD11b for KCs; CD11b for DCs, CD19 for B lymphocytes; CD4 and CD8 for T lymphocytes. An analysis by flow cytometry was performed to detect the presence of GFP⁺ cells. Approximately 20% of the Kupffer cells and the intrahepatic B lymphocytes, and between 5% and 10% of the dendritic cells and the T lymphocytes were GFP⁺ after the hydrodynamic injection of plasmid CAG GFP WPRE (Figure 3). Confirming the previous histochemical observations, no GFP⁺ Kupffer cells or other types of GFP⁺ non-parenchymal cells were detected by flow cytometry following the injection of the adeno-associated vector.

### Example 11. Blood glucose levels following the treatment with pCAG IGF-I

In order to examine whether the expression of exogenous IGF-I in the liver could prevent DT1, Tg IFNβ mice were treated on a weekly basis with a plasmid that expressed IGF-I under the control of the ubiquitous CAG promoter. The Tg IFNβ mice expressed human β interferon in pancreatic β cells. These mice develop manifest diabetes with lymphocyte infiltration of the islets when they are treated with very low doses of streptozotocin, which does not induce diabetes in control mice (Casellas et al., 2006; Pelegrin et al., 1998). It has previously been demonstrated that the local expression of IGF-I in β cells prevents infiltration of the islets and the death of β cells in these Tg IFNβ mice (Casellas et al., 2006).

Experimental diabetes was induced by the administration of daily injections of 25 mg/kg of streptozotocin (STZ) for 5 consecutive days. Seven days after the first administration of STZ, a plasmid that directed the expression of IGF-I under the control of the ubiquitous CAG promoter was hydrodynamically injected, once a week for 10 weeks. The experimental design is indicated in **Figure 4A****.**

Following the injection of the plasmid, the blood glucose levels were measured once a week for 16 weeks. Prior to the treatment with STZ, all the animals were normoglycemic. The animals that did not receive the treatment with STZ (Con) remained normoglycemic. Following the treatment with STZ, the blood glucose levels increased above 500 mg/dl in those animals treated with a non-coding plasmid (STZ-Con), whereas approximately 80% of the mice treated with STZ which received the weekly treatment with pIGF-I remained normoglycemic for the entire duration of the experiment, even after interrupting the treatment with IGF-I at week 10 post-STZ (**Figure 4B**).

When the incidence of diabetes was measured in the mice, it was discovered that 90% of the animals were diabetic at 8 weeks post-STZ in the group treated with non-coding plasmid (STZ-Con group), whereas 83% of the animals did not develop diabetes when they were treated with 5 µg of pCAG IGF-I (**Figure 4C**). An animal was considered to be diabetic when 2 consecutive glycemia measurements were greater than 250 mg/dl.

These results indicated that the hepatic expression of IGF-I following the hydrodynamic injection was capable of preventing the development of hyperglycemia in a mouse model of type 1 diabetes.

### Example 12. Analysis of lymphocyte infiltration of the pancreatic islets

Insulitis was measured 4 months after the treatment with STZ and the degree of insulitis in the normoglycemic mice treated with IGF-I was similar to that observed in the control mice not treated with STZ (**Figure 5A**).

Lymphocyte infiltration of the islets was also determined by flow cytometry. A relative quantification of the infiltrating lymphocytes (CD4⁺ T and CD8⁺ T) and macrophages (CD11b⁺) was performed 2 weeks after the first injection of STZ. A significant increase in CD4⁺ and CD8⁺ T cells was discovered in the pancreas of the STZ-Con mice as compared to the control animals not treated with STZ. In the IGF-I group, the number of infiltrating CD4⁺ and CD8⁺ T cells was significantly reduced as compared to the STZ-Con mice (**Figure 5B**).

Therefore, the islets of animals not treated with STZ had a normal insulin staining pattern, both at 15 days and at 4 months (**Figure 5C**).

Four months after the treatment with STZ, the animals treated with IGF-I presented a β cell mass similar to that of animals which had not been injected with STZ, whereas the STZ-Con mice showed a strong reduction, of approximately 90% (**Figure 6C**).

### Example 13. Analysis of the apoptosis and replication of β cells

In order to study whether the treatment with IGF-I protected β cells against STZ-induced apoptosis in Tg IFNβ mice, an immunohistochemical analysis was performed to detect non-β cells (with a cocktail of anti-glucagon, anti-somatostatin and anti-pancreatic polypeptide antibodies) and TUNEL was performed to identify apoptotic cells. Two weeks after the treatment with STZ, a 2.5-fold increase was detected in TUNEL-positive β cells in the STZ-Con mice as compared to the animals treated with IGF-I which had been injected with STZ (**Figure 6A**). This result confirmed that the treatment with IGF-I protected pancreatic β cells from the death mediated by the immune system.

Moreover, in order to determine whether the treatment with IGF-I induced replication of β cells in animals treated with STZ, a double immunohistochemical analysis for insulin and the Ki67 replication marker was performed. Two weeks after the injection of STZ, a two-fold increase was observed in β cells undergoing replication in the islets of the mice treated with IGF-I which had been injected with STZ, as compared to the STZ-Con animals (**Figure 6B**).

The increase in β cells undergoing replication following the treatment with IGF-I as compared to the STZ-Con mice suggested that the autoimmunity against β cells was blocked (Nir et al., 2007).

### Example 14. Measurement of the β cell mass in mice treated with IGF-I

The low levels of insulitis detected in the animals treated with IGF-I suggested that the treatment was causing an interruption of the autoimmune attack against β cells. An immunohistochemical detection of insulin was performed in pancreatic sections of Tg IFNβ mice 4 months after the treatment with STZ in order to determine whether the β cell mass was maintained in the animals treated with IGF-I (**Figure 6C**). Few islets could be detected in the pancreas of the STZ-Con mice and most of them were small and highly infiltrated. On the contrary, normoglycemic mice treated with IGF-I had a normal number of islets with a normal appearance at 16 weeks after the treatment with STZ.

### Example 15. Blood levels of insulin and IGF-I in mice treated with IGF-I

The capacity to maintain normoglycemia following the administration of STZ, the interruption of the progression of insulitis and the maintenance of the β cell mass in the animals treated with IGF-I suggested that their pancreas was functional and capable of producing appropriate blood levels of insulin. In order to confirm this, the serum levels of insulin were measured. The animals treated with the void plasmid (STZ-Con) presented approximately 80% lower serum levels of insulin, whereas the controls not treated with STZ (Con) remained normoinsulinaemic (**Figure 6D**). The animals treated with 5 µg of plasmid IGF-I that were capable of maintaining normoglycemia presented normal levels of insulin. The small percentage of animals treated with IGF-I that developed hyperglycemia had levels of insulin similar to those of the STZ-Con animals (the data are not shown).

In order to examine whether an increase in the circulating levels of IGF-I following an HTV injection of pIGF-I could be responsible for the protection against the development of hyperglycemia observed, the levels of total circulating IGF-I were measured 8 weeks after the induction of diabetes. However, no differences in the circulating levels of IGF-I were detected between the groups (**Figure 6E**).

### Example 16. The specific overexpression of exogenous IGF-I in hepatocytes does not lead to the prevention of diabetes

As described above, the CAG promoter is capable of directing the expression of the transgene in hepatocytes and also in several non-parenchymal hepatic cells following the hydrodynamic injection process. In order to further characterise whether the protection against the development of diabetes following the treatment with IGF-I depended on the specific expression of exogenous IGF-I in hepatocytes, a new plasmid construct was examined which expressed IGF-I under the control of a hepatocyte-specific promoter composed of the hepatocyte control region (HCR) enhancer of apolipoprotein E and the human α-antitrypsin promoter (phAAT-IGF). As previously demonstrated in the study, the hAAT promoter is as potent as the GAC promoter in terms of transcriptional activity.

The expression of IGF-I in the liver of the mice that were injected with the plasmid was determined by means of RT-PCR, using specific primers for exogenous IGF-I. Twenty-four hours after the HTV injection of phAAT IGF-I, the expression of IGF-I from the plasmid could only be detected in the animals which had been injected with pCAG IGF-I WPRE (**Figure 7A**).

The IFNβ mice were treated with daily injections of 25 mg/kg of streptozotocin (STZ) for 5 consecutive days. Following the injection of STZ, a plasmid which directed the expression of IGF-I under the control of the hAAT hepatocyte-specific promoter (STZ-hAAT-IGF-I) was hydrodynamically injected every week for 4 weeks. Mice not treated with STZ and STZ-Con mice were included as controls. Two weeks after the injection of STZ, the cumulative incidence of diabetes was approximately 80% in the STZ-Con mice. The animals that expressed exogenous IGF-I under the control of the hAAT promoter did not show protection against diabetes and the cumulative incidence of the disease was similar to that of the STZ-Con mice **(****Figure 7B**). As expected, the animals not treated with STZ did not develop diabetes. Therefore, these results suggested that the hepatocyte-specific expression of exogenous IGF-I was not sufficient to prevent the development of diabetes in mice treated with STZ.

### Example 17. The overexpression of exogenous IGF-I under the control of the CD68 promoter in non-parenchymal hepatic cells is sufficient for the prevention of diabetes

Since the expression of plasmid IGF-I limited to hepatocytes was incapable of suppressing the onset of diabetes, the effects of the expression of IGF-I in other types of hepatic cells was examined. In the liver, the population of non-parenchymal cells is primarily made up of sinusoidal endothelial cells (LSEC), resident tissue macrophages (Kupffer cells), dendritic cells, astrocytes and liver-associated lymphocytes (Blouin et al., 1977). Only recently has it been demonstrated that many liver functions are dependent on the co-operation between non-parenchymal and parenchymal hepatic cells (Kmiec, 2001). Therefore, in order to examine whether the overexpression of exogenous IGF-I in non-parenchymal hepatic cells could lead to a reduction in hyperglycemia, a plasmid was generated which expressed IGF-I under the control of the murine CD68 promoter. CD68 is a macrophage marker and a type I transmembrane protein that is expressed in the endosomal compartment of all the cells of the mononuclear phagocyte lineage, including monocytes, macrophages, microglia, osteoclasts and, to a lesser extent, immature dendritic cells (Gough et al., 2001). Hepatic astrocytes cells also express CD68 (Viñas et al., 2003).

The same experimental design indicated in Figure 4A was followed. Tg IFNβ mice were treated with daily injections of 25 mg/kg of streptozotocin (STZ) for 5 consecutive days. Following the induction of diabetes, they were hydrodynamically injected once a week with 5 µg of a plasmid that directed the expression of IGF-I under the control of the murine CD68 promoter (STZ-CD68-IGF-I). One set of animals were treated with a void plasmid (STZ-Con), whereas another was not injected with STZ (Con). The expression of the plasmid was confirmed by means of PCR (**Figure 8A**). None of the mice not treated with STZ developed hyperglycemia, whereas 90% of the animals in the STZ-Con group became diabetic at week 6 post-STZ. On the contrary, the percentage of diabetic animals in the STZ-CD68-IGF-I group was only approximately 40% 6 weeks after the induction of diabetes (**Figure 8B**).

These results indicated that the overexpression of IGF-I in non-parenchymal hepatic cells was sufficient to prevent the onset of the disease in approximately 60% of the mice treated with STZ. Therefore, non-parenchymal hepatic cells may be involved in the prevention of DT1 in the Tg IFNβ mouse model of type 1 diabetes.

### Example 18. Changes in the hepatic and pancreatic regulatory T cells after the treatment with IGF-I

It has been demonstrated that regulatory T cells control autoimmune physiological processes, such as autoimmune diabetes, and it has been demonstrated that variations in the proportions of T_{reg} in the periphery regulate the evolution towards the disease (Belghith et al., 2003). Therefore, a relative quantification of the percentage of intrahepatic, intrasplenic and intrapancreatic T_{reg} (CD4⁺ CD25⁺ GITR⁺ T cells) was performed 1 month after the first injection of STZ - after 4 hydrodynamic injections of pCAG IGF-I and pCD68 IGF-I.

A significant increase, of 60% of T_{reg}, was detected in the liver of the animals treated with both CAG IGF-I and CD68 IGF-I, as compared to the STZ Con mice (**Figure 9A**). The percentage of intrahepatic T_{reg} in untreated animals (Con) was the lowest amongst the groups, which indicates that the increase in the percentage of T_{reg} observed in the groups treated with IGF-I as compared to STZ Con could not be exclusively attributed to the normoglycemic condition of the former.

Moreover, an approximately two-fold increase in the levels of intrapancreatic T_{reg} was detected in the animals treated with both CAG IGF-I and CD68 IGF-I, as compared to the STZ Con mice (Figure 9B). Finally, all the groups treated with STZ showed similar percentages of intrasplenic T_{reg} (**Figure 9C**).

These data suggested that the treatment with IGF-I induced a local increase in the percentage of intrahepatic and intrapancreatic T_{reg} that could explain the protection against the development of diabetic hyperglycemia observed.

An increase in the percentage of T_{reg} in the liver of the animals treated with pIGF-I one month after the treatment with STZ (Figure 9A) suggested that this cell population could be responsible for the control of autoimmunity in the Tg IFNβ model of DT1. Therefore, the contribution of regulatory T cells to the development of diabetes in the Tg IFNβ model was determined by the reduction of this cell population.

A depletion of CD25⁺ cells was performed at days 9 and 12 by means of intraperitoneal injections of rat anti-mouse CD25 monoclonal antibody at a dose of 30 µg/mouse (≈1.5 mg/kg). The control mice received rabbit serum.

The cumulative incidence of diabetes in all the groups is shown in **Figure 9D****.** The animals that did not receive an STZ injection or treatment with a plasmid (Con group) did not develop diabetes (0 of 6 mice). On the contrary, over 80% (5/6) of the mice treated with STZ that received a hydrodynamic injection of a void plasmid (STZ group) were diabetic 3 weeks after the administration of STZ. The treatment with IGF-I in the mice treated with STZ that received rabbit serum as a control (STZ IGF-I group) reduced the induction of diabetes to 33% of the animals (2 of 6), which shows a protective effect against the development of hyperglycemia that has been previously described in this study. However, the depletion of CD25⁺ lymphocytes in the mice treated with pIGF-I which were injected with STZ (STZ-CD25 IGF-I) increased the onset of diabetes by up to 100% (5 of 5 animals), 2 weeks after the depletion regime, thereby completely suppressing the protective phenotype observed in the STZ IGF-I group. Similarly, the percentage of diabetic animals when the depletion was performed in animals treated with the void plasmid that were injected with STZ was 80%.

These results indicated that the depletion of T_{reg} in the autoimmune diabetes Tg IFNβ mouse model could be breaking the peripheral tolerance to β cells, leading to the onset of the disease. This also suggested that T_{reg} cells could be involved in the IGF-I-mediated protection against DT1.

### Example 19. The muscular electrotransfer of pCMV-IGF-I does not prevent Type 1 Diabetes

In another set of experiments, an experimental diabetic state was induced in RIP-I/hIFNβ mice by administering daily injections of 25 mg/kg of streptozotocin (STZ) for 5 consecutive days. Following the induction of diabetes, a plasmid was electrotransferred which directs the expression of IGF-I, under the control of the ubiquitous CMV promoter (pCMV IGF-I), to the muscles of transgenic IFNβ mice previously treated with STZ (STZ IGF-I). As controls, untreated mice (Con) and mice which had been injected with STZ (STZ-Con) were used. As expected, forty days after the induction of diabetes, none of the animals (0/6) in the Con group had become diabetic, whereas 80% of the mice in the STZ-Con group (4/5) developed hyperglycemia. All the STZ-IGF-I animals (7/7) became diabetic. The animals that developed hyperglycemia showed evident symptoms of diabetes, such as a severe lymphocyte infiltration in the pancreas (insulitis) and an 80% reduction in the levels of circulating insulin as compared to the control animals. These results indicated that the electrotransfer of a plasmid that encodes IGF-I in the muscles of pre-diabetic transgenic IFNβ mice was not capable of protecting against the disease.

### BIBLIOGRAPHY

1. Aliño, S.F., Herrero, M.J., Noguera, I., Dasí, F., and Sánchez, M. (2007). Pig liver gene therapy by noninvasive interventionist catheterism. Gene Ther 14, 334-343.
2. Allenbach, Y., Solly, S., Gregoire, S., Dubourg, O., Salomon, B., Butler-Browne, G., Musset, L., Herson, S., Klatzmann, D., and Benveniste, O. (2009). Role of regulatory T cells in a new mouse model of experimental autoimmune myositis. Am J Pathol 174, 989-998.
3. Atkinson, M.A., and Eisenbarth, G.S. (2001). Type 1 diabetes: new perspectives on disease pathogenesis and treatment. Lancet 358, 221-229.
4. Banica, L., Besliu, A., Pistol, G., Stavaru, C., lonescu, R., Forsea, A.M., Tanaseanu, C., Dumitrache, S., Otelea, D., Tamsulea, I., et al. (2009). Quantification and molecular characterization of regulatory T cells in connective tissue diseases. Autoimmunity 42, 41-49.
5. Bergerot, I., Fabien, N., Maguer, V., and Thivolet, C. (1995). Insulin-like growth factor-1 (IGF-I) protects NOD mice from insulitis and diabetes. Clin Exp Immunol 102, 335-340.
6. Boyer, O., Saadoun, D., Abriol, J., Dodille, M., Piette, J.C., Cacoub, P., and Klatzmann, D. (2004). CD4+CD25+ regulatory T-cell deficiency in patients with hepatitis C-mixed cryoglobulinemia vasculitis. Blood 103, 3428-3430.
7. Bradshaw, G., Gutierrez, A., Miyake, J.H., Davis, K.R., Li, A.C., Glass, C.K., Curtiss, L.K., and Davis, R.A. Facilitated replacement of Kupffer cells expressing a paraoxonase-1 transgene is essential for ameliorating atherosclerosis in mice. Proc Natl Acad Sci USA (2005) vol. 102 (31) pp. 11029-34
8. Cao, D., Malmstrom, V., Baecher-Allan, C., Hafler, D., Klareskog, L., and Trollmo, C. (2003). Isolation and functional characterization of regulatory CD25brightCD4+ T cells from the target organ of patients with rheumatoid arthritis. Eur J Immunol 33, 215-223.
9. Casellas, A., Salavert, A., Agudo, J., Ayuso, E., Jimenez, V., Moya, M., Muñoz, S., Franckhauser, S., and Bosch, F. (2006). Expression of IGF-I in pancreatic islets prevents lymphocytic infiltration and protects mice from type 1 diabetes. Diabetes 55, 3246-3255.
10. Cohen, J.L., and Boyer, O. (2006). The role of CD4+CD25hi regulatory T cells in the physiopathogeny of graft-versus-host disease. Curr Opin Immunol 18, 580-585.
11. Couper, J.J., Hudson, I., Werther, G.A., Warne, G.L., Court, J.M., and Harrison, L.C. (1991). Factors predicting residual beta-cell function in the first year after diagnosis of childhood type 1 diabetes. Diabetes Res Clin Pract 11, 9-16.
12. Chen, L., Yang, P., Zhou, H., He, H., Ren, X., Chi, W., Wang, L., and Kijlstra, A. (2008). Diminished frequency and function of CD4+CD25high regulatory T cells associated with active uveitis in Vogt-Koyanagi-Harada syndrome. Invest Ophthalmol Vis Sci 49, 3475-3482.
13. Chen, Z., Herman, A.E., Matos, M., Mathis, D., and Benoist, C. (2005). Where CD4+CD25+ T reg cells impinge on autoimmune diabetes. J Exp Med 202, 1387-1397.
14. Denning, T.L., Kim, G., and Kronenberg, M. (2005). Cutting edge: CD4+CD25+ regulatory T cells impaired for intestinal homing can prevent colitis. J Immunol 174, 7487-7491.
15. Eizirik, D.L., Colli, M.L., and Ortis, F. (2009). The role of inflammation in insulitis and beta-cell loss in type 1 diabetes. Nat Rev Endocrinol 5, 219-226.
16. Forger, F., Villiger, P.M., and Ostensen, M. (2009). Pregnancy in patients with ankylosing spondylitis: do regulatory T cells play a role? Arthritis Rheum 61, 279-283.
17. George, M., Ayuso, E., Casellas, A., Costa, C., Devedjian, J.C., and Bosch, F. (2002). Beta cell expression of IGF-I leads to recovery from type 1 diabetes. J Clin Invest 109, 1153-1163.
18. George, J. (2008). Mechanisms of disease: the evolving role of regulatory T cells in atherosclerosis. Nat Clin Pract Cardiovasc Med 5, 531-540.
19. Giannoukakis, N., Mi, Z., Rudert, W.A., Gambotto, A., Trucco, M., and Robbins, P. (2000). Prevention of beta cell dysfunction and apoptosis activation in human islets by adenoviral gene transfer of the insulin-like growth factor I. Gene Ther 7, 2015-2022.
20. Haas, J., Hug, A., Viehover, A., Fritzsching, B., Falk, C.S., Filser, A., Vetter, T., Milkova, L., Korporal, M., Fritz, B., et al. (2005). Reduced suppressive effect of CD4+CD25high regulatory T cells on the T cell immune response against myelin oligodendrocyte glycoprotein in patients with multiple sclerosis. Eur J Immunol 35, 3343-3352.
21. Holmen, N., Lundgren, A., Lundin, S., Bergin, A.M., Rudin, A., Sjovall, H., and Ohman, L. (2006). Functional CD4+CD25high regulatory T cells are enriched in the colonic mucosa of patients with active ulcerative colitis and increase with disease activity. Inflamm Bowel Dis 12, 447-456.
22. Janssen, J.A. (2008). Advantages and disadvantages of GH/IGF-I combination treatment. Reviews in endocrine & metabolic disorders.
23. Kaino, Y., Hirai, H., Ito, T., and Kida, K. (1996). Insulin-like growth factor I (IGF-I) delays the onset of diabetes in nonobese diabetic (NOD) mice. Diabetes Res Clin Pract 34, 7-11.
24. Klein, I., Cornejo, J.C., Polakos, N.K., John, B., Wuensch, S.A., Topham, D.J., Pierce, R.H., and Crispe, I.N. (2007). Kupffer cell heterogeneity: functional properties of bone marrow derived and sessile hepatic macrophages. Blood 110, 4077-4085.
25. Kriegel, M.A., Lohmann, T., Gabler, C., Blank, N., Kalden, J.R., and Lorenz, H.M. (2004). Defective suppressor function of human CD4+ CD25+ regulatory T cells in autoimmune polyglandular syndrome type II. J Exp Med 199, 1285-1291.
26. Kuhn, A., Beissert, S., and Krammer, P.H. (2009). CD4(+)CD25 (+) regulatory T cells in human lupus erythematosus. Arch Dermatol Res 301, 71-81.
27. Kukreja, A., Cost, G., Marker, J., Zhang, C., Sun, Z., Lin-Su, K., Ten, S., Sanz, M., Exley, M., Wilson, B., et al. (2002). Multiple immuno-regulatory defects in type-1 diabetes. J Clin Invest 109, 131-140.
28. Lan, R.Y., Cheng, C., Lian, Z.X., Tsuneyama, K., Yang, G.X., Moritoki, Y., Chuang, Y.H., Nakamura, T., Saito, S., Shimoda, S., et al. (2006). Liver-targeted and peripheral blood alterations of regulatory T cells in primary biliary cirrhosis. Hepatology 43, 729-737.
29. Lindley, S., Dayan, C.M., Bishop, A., Roep, B.O., Peakman, M., and Tree, T.I. (2005). Defective suppressor function in CD4(+)CD25(+) T-cells from patients with type 1 diabetes. Diabetes 54, 92-99.
30. Liu, F., Song, Y., and Liu, D. (1999). Hydrodynamics-based transfection in animals by systemic administration of plasmid DNA. Gene Ther 6, 1258-1266.
31. Luth, S., Huber, S., Schramm, C., Buch, T., Zander, S., Stadelmann, C., Bruck, W., Wraith, D.C., Herkel, J., and Lohse, A.W. (2008). Ectopic expression of neural autoantigen in mouse liver suppresses experimental autoimmune neuroinflammation by inducing antigen-specific Tregs. J Clin Invest 118, 3403-3410.
32. Matta, B., Jha, P., Bora, P.S., and Bora, N.S. (2008). Tolerance to melanin-associated antigen in autoimmune uveitis is mediated by CD4+CD25+ T-regulatory cells. Am J Pathol 173, 1440-1454.
33. McHugh, R.S. (2005). Autoimmune gastritis is a well-defined autoimmune disease model for the study of CD4+CD25+ T cell-mediated suppression. Curr Top Microbiol Immunol 293, 153-177.
34. Meriggioli, M.N., Sheng, J.R., Li, L., and Prabhakar, B.S. (2008). Strategies for treating autoimmunity: novel insights from experimental myasthenia gravis. Ann N Y Acad Sci 1132, 276-282.
35. Pan, D., Shin, Y.H., Gopalakrishnan, G., Hennessey, J., and De Groot, L.J. (2009). Regulatory T cells in Graves' disease. Clin Endocrinol (Oxf).
36. Pelegrin, M., Devedjian, J.C., Costa, C., Visa, J., Solanes, G., Pujol, A., Asins, G., Valera, A., and Bosch, F. (1998). Evidence from transgenic mice that interferon-beta may be involved in the onset of diabetes mellitus. J Biol Chem 273, 12332-12340.
37. Ricciardelli, I., Lindley, K.J., Londei, M., and Quaratino, S. (2008). Anti tumour necrosis-alpha therapy increases the number of FOXP3 regulatory T cells in children affected by Crohn's disease. Immunology 125, 178-183.
38. Senechal, B., Elain, G., Jeziorski, E., Grondin, V., Patey-Mariaud de Serre, N., Jaubert, F., Beldjord, K., Lellouch, A., Glorion, C., Zerah, M., et al. (2007). Expansion of regulatory T cells in patients with Langerhans cell histiocytosis. PLoS Med 4, e253.
39. Sheng, J.R., Li, L.C., Ganesh, B.B., Prabhakar, B.S., and Meriggioli, M.N. (2008). Regulatory T cells induced by GM-CSF suppress ongoing experimental myasthenia gravis. Clin Immunol 128, 172-180.
40. Sugiyama, H., Gyulai, R., Toichi, E., Garaczi, E., Shimada, S., Stevens, S.R., McCormick, T.S., and Cooper, K.D. (2005). Dysfunctional blood and target tissue CD4+CD25high regulatory T cells in psoriasis: mechanism underlying unrestrained pathogenic effector T cell proliferation. J Immunol 174, 164-173.
41. Uesugi, T., Froh, M., Arteel, G.E., Bradford, B.U., Gäbele, E., Wheeler, M.D., and Thurman, R.G.Delivery of IkappaB superrepressor gene with adenovirus reduces early alcohol-induced liver injury in rats. Hepatology (2001) vol. 34 (6) pp. 1149-57.
42. Valencia, X., Yarboro, C., Illei, G., and Lipsky, P.E. (2007). Deficient CD4+CD25high T regulatory cell function in patients with active systemic lupus erythematosus. J Immunol 178, 2579-2588.
43. van Til, N.P., Markusic, D.M., van der Rijt, R., Kunne, C., Hiralall, J.K., Vreeling, H., Frederiks, W.M., Oude-Elferink, R.P., and Seppen, J. Kupffer cells and not liver sinusoidal endothelial cells prevent lentiviral transduction of hepatocytes. Mol Ther (2005) vol. 11 (1) pp. 26-34.
44. Vergani, D., and Mieli-Vergani, G. (2008). Aetiopathogenesis of autoimmune hepatitis. World J Gastroenterol 14, 3306-3312.
45. Viglietta, V., Baecher-Allan, C., Weiner, H.L., and Hafler, D.A. (2004). Loss of functional suppression by CD4+CD25+ regulatory T cells in patients with multiple sclerosis. J Exp Med 199, 971-979.
46. Yu, Q., Que, L.G., and Rockey, D.C. Adenovirus-mediated gene transfer to nonparenchymal cells in normal and injured liver. Am J Physiol Gastrointest Liver Physiol (2002) vol. 282 (3) pp. G565-72.

## Claims

1. A DNA construct which consists of a nucleotide sequence that encodes insulin-like growth factor I (IGF-I), or a functional biological equivalent thereof, **characterised in that** it is included in an adequate vector to express IGF-I in hepatic cells.

2. A DNA construct according to claim 1, wherein the hepatic cells are non-parenchymal hepatic cells (NPC).

3. A DNA construct according to claim 2, wherein said vector consists of a plasmid that comprises an adequate promoter to express IGF-I, or a functional biological equivalent thereof, in NPCs.

4. A DNA construct according to claim 3, wherein said vector consists of a plasmid that comprises an adequate promoter to express IGF-I, or a functional biological equivalent thereof, in Kupffer cells.

5. A DNA construct according to claims 3 or 4, wherein the promoter is selected from: CAG or CD68.

6. A DNA construct according to any of claims 3 to 5, which consists of plasmid pCAG IGF-I WPRE, **characterised by** SEQ ID No: 1.

7. A DNA construct according to any of claims 3 to 5, which consists of plasmid pCD68 IGF-I, **characterised by** SEQ ID No: 2.

8. Use of the DNA construct according to any of claims 1 to 7, to manufacture an active pharmaceutical composition to prevent or treat autoimmune diseases.

9. Use of the DNA construct according to claim 8, wherein the autoimmune diseases are caused by the dysfunction or de-regulation of T_{reg} cells.

10. Use according to claims 8 or 9, wherein the autoimmune disease is Type 1 Diabetes (T1D).

11. Pharmaceutical compositions that comprise the DNA construct according to claims 1 to 7 and a pharmaceutically acceptable vehicle.

12. A DNA construct that consists of a nucleotide sequence that encodes an insulin-like growth factor 1 (IGF-I), or a functional biological equivalent thereof, included in an adequate vector to express IGF-I or a functional biological equivalent thereof in hepatic cells, to be used in the prevention or treatment of autoimmune diseases.

13. A DNA construct according to claim 12, wherein the hepatic cells are non-parenchymal hepatic cells (NPC).

14. A DNA construct according to claims 12 or 13, wherein the autoimmune diseases are caused by a dysfunction or de-regulation of T_{reg} cells.

15. A DNA construct according to claims 12 to 14, wherein the autoimmune disease is Type I Diabetes (T1D).

16. A DNA construct according to claims 12 or 13, which consists of plasmid pCAG IGF-I WPRE, represented by SEQ ID No: 1.

17. A DNA construct according to claims 12 or 13, which consists of plasmid pCD68 IGF-I, represented by SEQ ID No: 2.

18. A method for the prevention or treatment of autoimmune diseases that comprises administering a therapeutically effective dose of the pharmaceutical composition of claim 11 to a patient.

19. A method according to claim 18, wherein the autoimmune diseases are caused by a dysfunction or de-regulation of T_{reg} cells.

20. A method according to claims 18 or 19, wherein the autoimmune disease is Type 1 Diabetes (T1D).

21. A method according to claims 18 to 20, **characterised in that** the composition is directly administered to the patient's liver by means of a local injection.


SECUENCE LISTING

<110>  UNIVERSIDAD AUTÒNOMA DE BARCELONA

<120>   Gene therapy compositions for the prevention and/or treatment of
autoimmune diseases

<130>  EP-05064

<160>  6

<170>  PatentIn version 3.5

<210>  1
<211>  6407
<212>  DNA
<213>  Artificial

<220>
<223>  IGF-I-coding plasmid under the control of the CAG hybrid promoter

<220>
<221>  promoter
<222>  (1)..(645)
<223>  CAG hybrid promoter

<220>
<221>  Intron
<222>  (646)..(1611)
<223>  Chicken beta-actin intron

<220>
<221>  CDS
<222>  (1805)..(2500)
<223>  Murine IGF-I

<220>
<221>  polyA signal
<222>  (3341)..(3473)
<223>  SV40

<220>
<221>  characteristic
<222>  (3596)..(3194)
<223>  WPRE

<220>
<221>  characteristic
<222>  (4313)..(5170)
<223>  bla gene, antibiotic-resistant

<400>  1
ctagttatta atagtaatca attacggggt cattagttca tagcccatat atggagttcc      60

gcgttacata acttacggta aatggcccgc ctggctgacc gcccaacgac ccccgcccat     120

tgacgtcaat aatgacgtat gttcccatag taacgccaat agggactttc cattgacgtc     180

aatgggtgga gtatttacgg taaactgccc acttggcagt acatcaagtg tatcatatgc     240

caagtacgcc ccctattgac gtcaatgacg gtaaatggcc cgcctggcat tatgcccagt     300

acatgacctt atgggacttt cctacttggc agtacatcta cgtattagtc atcgctatta     360

ccatggtcga ggtgagcccc acgttctgct tcactctccc catctccccc ccctccccac     420

ccccaatttt gtatttattt atttttaat tattttgtgc agcgatgggg gcgggggggg 480

ggggggggcg cgcgccaggc ggggcggggc ggggcgaggg gcggggcggg gcgaggcgga 540

gaggtgcggc ggcagccaat cagagcggcg cgctccgaaa gtttcctttt atggcgaggc 600

ggcggcggcg gcggccctat aaaaagcgaa gcgcgcggcg ggcgggagtc gctgcgttgc 660

cttcgccccg tgccccgctc cgcgccgcct cgcgccgccc gccccggctc tgactgaccg 720

cgttactccc acaggtgagc gggcgggacg gcccttctcc tccgggctgt aattagcgct 780

tggtttaatg acggcttgtt tcttttctgt ggctgcgtga aagccttgag gggctccggg 840

agggcccttt gtgcgggggg agcggctcgg ggggtgcgtg cgtgtgtgtg tgcgtgggga 900

gcgccgcgtg cggctccgcg ctgcccggcg gctgtgagcg ctgcgggcgc ggcgcggggc 960

tttgtgcgct ccgcagtgtg cgcgagggga gcgcggccgg gggcggtgcc ccgcggtgcg 1020

gggggctgcg aggggaacaa aggctgcgtg cggggtgtgt gcgtgggggg gtgagcaggg 1080

ggtgtgggcg cgtcggtcgg gctgcaaccc cccctgcacc cccctccccg agttgctgag 1140

cacggcccgg cttcgggtgc ggggctccgt acggggcgtg gcgcggggct cgccgtgccg 1200

ggcggggggt ggcggcaggt gggggtgccg ggcggggcgg ggccgcctcg ggccggggag 1260

ggctcggggg aggggcgcgg cggcccccgg agcgccggcg gctgtcgagg cgcggcgagc 1320

cgcagccatt gccttttatg gtaatcgtgc gagagggcgc agggacttcc tttgtcccaa 1380

atctgtgcgg agccgaaatc tgggaggcgc cgccgcaccc cctctagcgg gcgcggggcg 1440

aagcggtgcg gcgccggcag gaaggaaatg ggcggggagg gccttcgtgc gtcgccgcgc 1500

cgccgtcccc ttctccctct ccagcctcgg ggctgtccgc ggggggacgg ctgccttcgg 1560

gggggacggg gcagggcggg gttcggcttc tggcgtgtga ccggcggctc tagagcctct 1620

gctaaccatg ttcatgcctt cttctttttc ctacagctcc tgggcaacgt gctggttatt 1680

gtgctgtctc atcattttgg caaagaattg attaattcga gcgaacgcgt cgagtcgctc 1740

ggtacgattt aaattgaatt ggcctcgagg tcgacggtat cgataagctt gatatcgaat 1800

tccg tag atg ctt tca caa acc cca ccc aca aaa caa cac atg ttc tta 1849
Met Leu Ser Gln Thr Pro Pro Thr Lys Gln His Met Phe Leu
1               5                   10

agt cct cag ttt tgt gtt cac ctc ggc ctc ata gta ccc act ctg acc 1897
Ser Pro Gln Phe Cys Val His Leu Gly Leu Ile Val Pro Thr Leu Thr
15                  20                  25                  30

tgc tgt gta aac gac ccg gac cta cca aaa tga ccg cac ctg caa taa 1945
Cys Cys Val Asn Asp Pro Asp Leu Pro Lys Pro His Leu Gln
                35                  40

aga tac aca tca tgt cgt ctt cac acc tct tct acc tgg cgc tct gct 1993
Arg Tyr Thr Ser Cys Arg Leu His Thr Ser Ser Thr Trp Arg Ser Ala
45                  50                  55                  60

tgc tca cct tca cca gct cca cca cag ctg gac cag aga ccc ttt gcg 2041
Cys Ser Pro Ser Pro Ala Pro Pro Gln Leu Asp Gln Arg Pro Phe Ala

                65                  70                  75

ggg ctg agc tgg tgg atg ctc ttc agt tcg tgt gtg gac cga ggg gct 2089
Gly Leu Ser Trp Trp Met Leu Phe Ser Ser Cys Val Asp Arg Gly Ala
            80                  85                  90

ttt act tca aca agc cca cag gct atg gct cca gca ttc gga ggg cac 2137
Phe Thr Ser Thr Ser Pro Gln Ala Met Ala Pro Ala Phe Gly Gly His
        95                  100                 105

ctc aga cag gca ttg tgg atg agt gtt gct tcc gga gct gtg atc tga 2185
Leu Arg Gln Ala Leu Trp Met Ser Val Ala Ser Gly Ala Val Ile
    110                 115                 120

gga gac tgg aga tgt act gtg ccc cac tga agc cta caa aag cag ccc 2233
Gly Asp Trp Arg Cys Thr Val Pro His Ser Leu Gln Lys Gln Pro
    125                 130                 135

gct cta tcc gtg ccc agc gcc aca ctg aca tgc cca aga ctc aga agg 2281
Ala Leu Ser Val Pro Ser Ala Thr Leu Thr Cys Pro Arg Leu Arg Arg
    140                 145                 150

aag tac att tga aga aca caa gta gag gaa gtg cag gaa aca aga cct 2329
Lys Tyr Ile Arg Thr Gln Val Glu Glu Val Gln Glu Thr Arg Pro
155                 160                 165

aca gaa tgt agg agg agc ctc cca cgg agc aga aaa tgc cac atc acc 2377
Thr Glu Cys Arg Arg Ser Leu Pro Arg Ser Arg Lys Cys His Ile Thr
170                 175                 180                 185

gca gga tcc ttt gct gct tga gca acc tgc aaa aca tcg aaa cac cta 2425
Ala Gly Ser Phe Ala Ala Ala Thr Cys Lys Thr Ser Lys His Leu
                190                 195                 200

cca aat aac aat aat aag tcc aat aac att aca aag atg ggc att tcc 2473
Pro Asn Asn Asn Asn Lys Ser Asn Asn Ile Thr Lys Met Gly Ile Ser
                205                 210                 215

ccc aat gaa ata tac aag taa aca ttc cgaattcctg cagtggccat 2520
Pro Asn Glu Ile Tyr Lys Thr Phe
            220

tttttggctg ggcacccggg tcgccatcca aaaaatggcc aggatccact agttctagag 2580

cggccgcaag aattcccgat aatcaacctc tggattacaa aatttgtgaa agattgactg 2640

gtattcttaa ctatgttgct ccttttacgc tatgtggata cgctgcttta atgcctttgt 2700

atcatgctat tgcttcccgt atggctttca ttttctcctc cttgtataaa tcctggttgc 2760

tgtctcttta tgaggagttg tggcccgttg tcaggcaacg tggcgtggtg tgcactgtgt 2820

ttgctgacgc aacccccact ggttggggca ttgccaccac ctgtcagctc ctttccggga 2880

ctttcgcttt ccccctccct attgccacgg cggaactcat cgccgcctgc cttgcccgct 2940

gctggacagg ggctcggctg ttgggcactg acaattccgt ggtgttgtcg gggaagctga 3000

cgtcctttcc atggctgctc gcctgtgttg ccacctggat tctgcgcggg acgtccttct 3060

gctacgtccc ttcggccctc aatccagcgg accttccttc ccgcggcctg ctgccggctc 3120

tgcggcctct tccgcgtctt cgccttcgcc ctcagacgag tcggatctcc ctttgggccg 3180

cctccccgca tcgggaattc gagctcggta cccgggaatc aattcactcc tcaggtgcag 3240

gctgcctatc agaaggtggt ggctggtgtg gccaatgccc tggctcacaa ataccactga 3300

gatcggccgc ttccctttag tgagggttaa tgcttcgagc agacatgata agatacattg 3360

atgagtttgg acaaaccaca actagaatgc agtgaaaaaa atgctttatt tgtgaaattt 3420

gtgatgctat tgctttattt gtaaccatta taagctgcaa taaacaagtt aacaacaaca 3480

attcgatatc aagcttatcg ataccgtcga cctcgagggg gggcccggta cccaattcgc 3540

cctatagtga gtcgtattac gcgcgctcac tggccgtcgt tttacaacgt cgtgactggg 3600

aaaaccctgg cgttacccaa cttaatcgcc ttgcagcaca tccccctttc gccagctggc 3660

gtaatagcga agaggcccgc accgatcgcc cttcccaaca gttgcgcagc ctgaatggcg 3720

aatgggacgc gccctgtagc ggcgcattaa gcgcggcggg tgtggtggtt acgcgcagcg 3780

tgaccgctac acttgccagc gccctagcgc ccgctccttt cgctttcttc ccttcctttc 3840

tcgccacgtt cgccggcttt ccccgtcaag ctctaaatcg ggggctccct ttagggttcc 3900

gatttagtgc tttacggcac ctcgacccca aaaaacttga ttagggtgat ggttcacgta 3960

gtgggccatc gccctgatag acggtttttc gccctttgac gttggagtcc acgttcttta 4020

atagtggact cttgttccaa actggaacaa cactcaaccc tatctcggtc tattcttttg 4080

atttataagg gattttgccg atttcggcct attggttaaa aaatgagctg atttaacaaa 4140

aatttaacgc gaattttaac aaaatattaa cgcttacaat ttaggtggca cttttcgggg 4200

aaatgtgcgc ggaaccccta tttgtttatt tttctaaata cattcaaata tgtatccgct 4260

catgagacaa taaccctgat aaatgcttca ataatattga aaaaggaaga gtatgagtat 4320

tcaacatttc cgtgtcgccc ttattccctt ttttgcggca ttttgccttc ctgtttttgc 4380

tcacccagaa acgctggtga aagtaaaaga tgctgaagat cagttgggtg cacgagtggg 4440

ttacatcgaa ctggatctca acagcggtaa gatccttgag agttttcgcc ccgaagaacg 4500

ttttccaatg atgagcactt ttaaagttct gctatgtggc gcggtattat cccgtattga 4560

cgccgggcaa gagcaactcg gtaggcgcat acactattct cagaatgact tggttgagta 4620

ctcaccagtc acagaaaagc atcttacgga tggcatgaca gtaagagaat tatgcagtgc 4680

tgccataacc atgagtgata acactgcggc caacttactt ctgacaacga tcggaggacc 4740

gaaggagcta accgcttttt tgcacaacat gggggatcat gtaactcgcc ttgatcgttg 4800

ggaaccggag ctgaatgaag ccataccaaa cgacgagcgt gacaccacga tgcctgtagc 4860

aatggcaaca acgttgcgca aactattaac tggcgaacta cttactctag cttcccggca 4920

acaattaata gactggatgg aggcggataa agttgcagga ccacttctgc gctcggccct 4980

tccggctggc tggtttattg ctgataaatc tggagccggt gagcgtgggt ctcgcggtat 5040

cattgcagca ctggggccag atggtaagcc ctcccgtatc gtagttatct acacgacggg 5100

gagtcaggca actatggatg aacgaaatag acagatcgct gagataggtg cctcactgat 5160

taagcattgg taactgtcag accaagttta ctcatatata ctttagattg atttaaaact 5220

tcatttttaa tttaaaagga tctaggtgaa gatccttttt gataatctca tgaccaaaat 5280

cccttaacgt gagttttcgt tccactgagc gtcagacccc gtagaaaaga tcaaaggatc 5340

ttcttgagat cctttttttc tgcgcgtaat ctgctgcttg caaacaaaaa aaccaccgct 5400

accagcggtg gtttgtttgc cggatcaaga gctaccaact ctttttccga aggtaactgg 5460

cttcagcaga gcgcagatac caaatactgt ccttctagtg tagccgtagt taggccacca 5520

cttcaagaac tctgtagcac cgcctacata cctcgctctg ctaatcctgt taccagtggc 5580

tgctgccagt ggcgataagt cgtgtcttac cgggttggac tcaagacgat agttaccgga 5640

taaggcgcag cggtcgggct gaacgggggg ttcgtgcaca cagcccagct tggagcgaac 5700

gacctacacc gaactgagat acctacagcg tgagctatga gaaagcgcca cgcttcccga 5760

agggagaaag gcggacaggt atccggtaag cggcagggtc ggaacaggag agcgcacgag 5820

ggagcttcca gggggaaacg cctggtatct ttatagtcct gtcgggtttc gccacctctg 5880

acttgagcgt cgatttttgt gatgctcgtc aggggggcgg agcctatgga aaaacgccag 5940

caacgcggcc tttttacggt tcctggcctt ttgctggcct tttgctcaca tgttctttcc 6000

tgcgttatcc cctgattctg tggataaccg tattaccgcc tttgagtgag ctgataccgc 6060

tcgccgcagc cgaacgaccg agcgcagcga gtcagtgagc gaggaagcgg aagagcgccc 6120

aatacgcaaa ccgcctctcc ccgcgcgttg gccgattcat taatgcagct ggcacgacag 6180

gtttcccgac tggaaagcgg gcagtgagcg caacgcaatt aatgtgagtt agctcactca 6240

ttaggcaccc caggctttac actttatgct tccggctcgt atgttgtgtg gaattgtgag 6300

cggataacaa tttcacacag gaaacagcta tgaccatgat tacgccaagc gcgcaattaa 6360

ccctcactaa agggaacaaa agctggagct ccaccgcggt ggcggcc 6407

<210> 2
<211> 4749
<212> DNA
<213> Artificial

<220>
<223> Plasmid carrying the IGF-I gene expressed under the control of the
murine CD68 promoter.

<220>
<221> promoter
<222> (1)..(720)
<223> CD68 promoter

<220>
<221> CDS
<222> (1013)..(1396)
<223> CDS for IGF-I

<220>
<221> polyA signal
<222> (1674)..(1806)

<223>  SV40

<220>
<221>  characteristic
<222>  (2646)..(3506)
<223>  bla gene, resistance to ampicillin

<400>  2
ctagttgatt actgaatttg ccatgttgct tcctgcaata ccaaatgacc cacattacta      60

acatttggta atttgcctca gtgttaggca caagacatga gtgaaacttg ctgcattaag     120

acttaaactg gctgggtatg gtggcgcaag cctttaatcc cagcattctg ccggaggcag     180

aagcaggtgg atttctgagt tcgaggctag cctggtctac aaaagtgagt tccaggacag     240

ccaaggctac acaaaatacc tgtcttgaaa aaccaaaaaa aaaaaattca actggttggt     300

taggcctagc ccagatgatg taggtgtcaa ctcaccttgg ggatgtaggg agatggttgt     360

tctgtagctc ttcctgtcct gcaactaaat aaataggcta agctgacctc ttacatctgc     420

cttcggcctc ctgtgctagg attggagtgc atgggtgggg ttgcatttct taccaatgag     480

gaaagggctt cccatttctc tgctgcattg taagctccca agtagagcaa gttttgctta     540

ggtaagttcc gtgtgagtca gctgcctcat tctcacgagg taaccaaggc tttgtaccgc     600

ccactgagaa cgtcactgcc aatcacagcc taattgtgaa aaccaatggc ttgagtgggt     660

tgctaaagct gaggtgtctg agtcaggttt ggggtgggat tattttagtt aagggaagtg     720

aggcttttca tttcctcttc caagagaagg caaaggggat tggattgagg aaggaactgg     780

tgtagcctag ctggtctgag catctcttct agcctcgacc tcgaggtcga cggtatcgat     840

aagcttgata tcgaattccg aagatgcttt cacaaacccc acccacaaaa caacacatgt     900

tcttaagtcc tcagttttgt gttcacctcg gcctcatagt acccactctg acctgctgtg     960

taaacgaccc ggacctacca aaatgaccgc acctgcaata aagatacaca tc atg tcg    1018
                                                          Met Ser
                                                          1

tct tca cac ctc ttc tac ctg gcg ctc tgc ttg ctc acc ttc acc agc      1066
Ser Ser His Leu Phe Tyr Leu Ala Leu Cys Leu Leu Thr Phe Thr Ser
        5                   10                  15

tcc acc aca gct gga cca gag acc ctt tgc ggg gct gag ctg gtg gat      1114
Ser Thr Thr Ala Gly Pro Glu Thr Leu Cys Gly Ala Glu Leu Val Asp
    20                  25                  30

gct ctt cag ttc gtg tgt gga ccg agg ggc ttt tac ttc aac aag ccc      1162
Ala Leu Gln Phe Val Cys Gly Pro Arg Gly Phe Tyr Phe Asn Lys Pro
35                  40                  45                  50

aca ggc tat ggc tcc agc att cgg agg gca cct cag aca ggc att gtg      1210
Thr Gly Tyr Gly Ser Ser Ile Arg Arg Ala Pro Gln Thr Gly Ile Val
                55                  60                  65

gat gag tgt tgc ttc cgg agc tgt gat ctg agg aga ctg gag atg tac      1258
Asp Glu Cys Cys Phe Arg Ser Cys Asp Leu Arg Arg Leu Glu Met Tyr
            70                  75                  80

tgt gcc cca ctg aag cct aca aaa gca gcc cgc tct atc cgt gcc cag      1306

Cys Ala Pro Leu Lys Pro Thr Lys Ala Ala Arg Ser Ile Arg Ala Gln
        85                  90                  95

cgc cac act gac atg ccc aag act cag aag gaa gta cat ttg aag aac      1354
Arg His Thr Asp Met Pro Lys Thr Gln Lys Glu Val His Leu Lys Asn
    100                 105                 110

aca agt aga gga agt gca gga aac aag acc tac aga atg tag              1396
Thr Ser Arg Gly Ser Ala Gly Asn Lys Thr Tyr Arg Met
115                 120                 125

gaggagcctc ccacggagca gaaaatgcca catcaccgca ggatcctttg ctgcttgagc    1456

aacctgcaaa acatcgaaac acctaccaaa taacaataat aagtccaata acattacaaa    1516

gatgggcatt tcccccaatg aaatatacaa gtaaacattc cgaattcctg cagtggccat    1576

tttttggctg ggcacccggg tcgccatcca aaaaatggcc aggatccact agttctagag    1636

cggccgcttc cctttagtga gggttaatgc ttcgagcaga catgataaga tacattgatg    1696

agtttggaca aaccacaact agaatgcagt gaaaaaaatg ctttatttgt gaaatttgtg    1756

atgctattgc tttatttgta accattataa gctgcaataa acaagttaac aacaacaatt    1816

cgatatcaag cttatcgata ccgtcgacct cgaggggggg cccggtaccc aattcgccct    1876

atagtgagtc gtattacgcg cgctcactgg ccgtcgtttt acaacgtcgt gactgggaaa    1936

accctggcgt tacccaactt aatcgccttg cagcacatcc ccctttcgcc agctggcgta    1996

atagcgaaga ggcccgcacc gatcgccctt cccaacagtt gcgcagcctg aatggcgaat    2056

gggacgcgcc ctgtagcggc gcattaagcg cggcgggtgt ggtggttacg cgcagcgtga    2116

ccgctacact tgccagcgcc ctagcgcccg ctcctttcgc tttcttccct tcctttctcg    2176

ccacgttcgc cggctttccc cgtcaagctc taaatcgggg gctcccttta gggttccgat    2236

ttagtgcttt acggcacctc gaccccaaaa aacttgatta gggtgatggt tcacgtagtg    2296

ggccatcgcc ctgatagacg gtttttcgcc ctttgacgtt ggagtccacg ttctttaata    2356

gtggactctt gttccaaact ggaacaacac tcaaccctat ctcggtctat tcttttgatt    2416

tataagggat tttgccgatt tcggcctatt ggttaaaaaa tgagctgatt taacaaaaat    2476

ttaacgcgaa ttttaacaaa atattaacgc ttacaattta ggtggcactt ttcggggaaa    2536

tgtgcgcgga acccctattt gtttattttt ctaaatacat tcaaatatgt atccgctcat    2596

gagacaataa ccctgataaa tgcttcaata atattgaaaa aggaagagta tgagtattca    2656

acatttccgt gtcgccctta ttcccttttt tgcggcattt tgccttcctg tttttgctca    2716

cccagaaacg ctggtgaaag taaaagatgc tgaagatcag ttgggtgcac gagtgggtta    2776

catcgaactg gatctcaaca gcggtaagat ccttgagagt tttcgccccg aagaacgttt    2836

tccaatgatg agcactttta aagttctgct atgtggcgcg gtattatccc gtattgacgc    2896

cgggcaagag caactcggta ggcgcataca ctattctcag aatgacttgg ttgagtactc    2956

accagtcaca gaaaagcatc ttacggatgg catgacagta agagaattat gcagtgctgc    3016

cataaccatg agtgataaca ctgcggccaa cttacttctg acaacgatcg gaggaccgaa 3076

ggagctaacc gcttttttgc acaacatggg ggatcatgta actcgccttg atcgttggga 3136

accggagctg aatgaagcca taccaaacga cgagcgtgac accacgatgc ctgtagcaat 3196

ggcaacaacg ttgcgcaaac tattaactgg cgaactactt actctagctt cccggcaaca 3256

attaatagac tggatggagg cggataaagt tgcaggacca cttctgcgct cggcccttcc 3316

ggctggctgg tttattgctg ataaatctgg agccggtgag cgtgggtctc gcggtatcat 3376

tgcagcactg gggccagatg gtaagccctc ccgtatcgta gttatctaca cgacggggag 3436

tcaggcaact atggatgaac gaaatagaca gatcgctgag ataggtgcct cactgattaa 3496

gcattggtaa ctgtcagacc aagtttactc atatatactt tagattgatt taaaacttca 3556

tttttaattt aaaaggatct aggtgaagat cctttttgat aatctcatga ccaaaatccc 3616

ttaacgtgag ttttcgttcc actgagcgtc agaccccgta gaaaagatca aaggatcttc 3676

ttgagatcct ttttttctgc gcgtaatctg ctgcttgcaa acaaaaaaac caccgctacc 3736

agcggtggtt tgtttgccgg atcaagagct accaactctt tttccgaagg taactggctt 3796

cagcagagcg cagataccaa atactgtcct tctagtgtag ccgtagttag gccaccactt 3856

caagaactct gtagcaccgc ctacatacct cgctctgcta atcctgttac cagtggctgc 3916

tgccagtggc gataagtcgt gtcttaccgg gttggactca agacgatagt taccggataa 3976

ggcgcagcgg tcgggctgaa cggggggttc gtgcacacag cccagcttgg agcgaacgac 4036

ctacaccgaa ctgagatacc tacagcgtga gctatgagaa agcgccacgc ttcccgaagg 4096

gagaaaggcg gacaggtatc cggtaagcgg cagggtcgga acaggagagc gcacgaggga 4156

gcttccaggg ggaaacgcct ggtatcttta tagtcctgtc gggtttcgcc acctctgact 4216

tgagcgtcga tttttgtgat gctcgtcagg ggggcggagc ctatggaaaa acgccagcaa 4276

cgcggccttt ttacggttcc tggccttttg ctggcctttt gctcacatgt tctttcctgc 4336

gttatcccct gattctgtgg ataaccgtat taccgccttt gagtgagctg ataccgctcg 4396

ccgcagccga acgaccgagc gcagcgagtc agtgagcgag gaagcggaag agcgcccaat 4456

acgcaaaccg cctctccccg cgcgttggcc gattcattaa tgcagctggc acgacaggtt 4516

tcccgactgg aaagcgggca gtgagcgcaa cgcaattaat gtgagttagc tcactcatta 4576

ggcaccccag gctttacact ttatgcttcc ggctcgtatg ttgtgtggaa ttgtgagcgg 4636

ataacaattt cacacaggaa acagctatga ccatgattac gccaagcgcg caattaaccc 4696

tcactaaagg gaacaaaagc tggagctcca ccgcggtggc ggccgctcta gaa 4749

<210> 3
<211> 19
<212> DNA
<213> Artificial Sequence

<220>
<223>  GFP forward primer

<400>  3
aagttcatct gcaccaccg                                                    19


<210>  4
<211>  21
<212>  DNA
<213>  Artificial Sequence

<220>
<223>  GFP reverse primer

<400>  4
tccttgaaga agatggtgcg c                                                 21


<210>  5
<211>  21
<212>  DNA
<213>  Artificial Sequence

<220>
<223>  IGF-I forward primer

<400>  5
ctgggcaacg tgctggttat t                                                 21


<210>  6
<211>  24
<212>  DNA
<213>  Artificial Sequence

<220>
<223>  IGF-I reverse primer

<400>  6
gggtttgtga aagcatctac ggaa                                              24


1